# EUROPÄISCHE PATENTANMELDUNG

(11) **EP 2 450 400 A2**
(43) Veröffentlichungstag der Anmeldung: **09.05.2012**
(21) Anmeldenummer: 11192753.9
(22) Anmeldetag: 14.08.2009
(51) Int. Cl.: C08K 5/00, C08K 5/32, C07D 211/94

(54) **Stabilisatoren für unbelebte organische materialien**

(30) Priorität: 28.08.2008 EP 08163194
(62) Teilanmeldung aus: 09781835.5
(71) Anmelder: BASF SE, 67056 Ludwigshafen (DE)
(72) Erfinder: Glaser, Alban, 68199 Mannheim (DE); Haremza, Sylke, 69151 Neckargemünd (DE); Schambony, Simon, 67061 Ludwigshafen (DE)

(57) **Zusammenfassung**

Die vorliegende Erfindung betrifft die Verwendung sterisch gehinderte Amine, enthaltend eine oder mehere Gruppen der allgemeinen Forrmel (I) 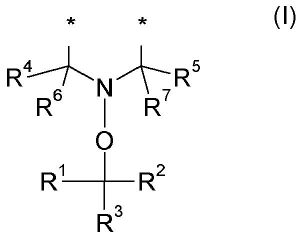 zur Stabilisierung von unbelebtem organischen Material, insbesondere von Kunststoffen oder Lacken, wobei, R¹, R² unabhängig voneinander, gleich oder verschieden, H, C₁-C₂₀-Alkyl, C₃-C₁₅-Cycloalkyl, Aryl, Hetaryl, Heterocyclus, R⁴, R⁵, R⁶, R⁷ unabhängig voneinander, gleich oder verschieden, C₁-C₂₀-Alkyl, oder R⁴ und R⁶ oder R⁵ und R⁷ zusammen eine Tetramethylen-oder Pentamethylengruppe sind, und R³ Aryl, Hetaryl ist. Weiterhin betrifft die Erfindung Verfahren zur Stabilisierung von unbelebtem organischen Material, insbesondere von Kunststoffen oder Lacken. Ein weiterer Gegenstand der Erfindung sind ausgewählte sterisch gehinderte Amine, enthaltend eine oder mahrere Gruppen der allgemeinen Formel (I). Ein weiterer Gegenstand der Erfindung sind Materialien, welche ausgewählte sterisch gehinderte Amine enthalten.

## Beschreibung

Die vorliegende Erfindung betrifft die Verwendung sterisch gehinderte Amine, enthaltend eine oder mehrere Gruppen der allgemeinen Formel (I) 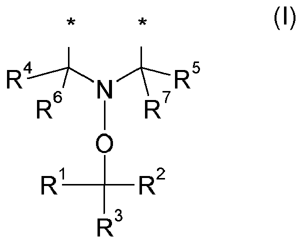 zur Stabilisierung von unbelebtem organischen Material, insbesondere von Kunststoffen oder Lacken,
wobei,
- R¹, R²: unabhängig voneinander, gleich oder verschieden, H, C₁-C₂₀-Alkyl, C₃-C₁₅-Cycloalkyl, Aryl, Hetaryl, Heterocyclus,
- R⁴, R⁵, R⁶, R⁷: unabhängig voneinander, gleich oder verschieden, C₁-C₂₀-Alkyl, oder R⁴ und R⁶ oder R⁵ und R⁷ zusammen eine Tetramethylen- oder Pentamethylengruppe
sind, und
- R³: Aryl, Hetaryl
ist, und
wobei R¹ bis R⁷ jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch Hydroxy, Amino, Mono- oder Di-C₁-C₂₀-Alkylamino, Nitro, Cyano, CO₂M¹, CONM¹M², SO₃M¹, C₁-C₂₀-Alkyl, C₁-C₂₀-Alkoxy, Aryl, Heterocyclus, Heteroatome oder Halogen substituiert sein können, wobei diese ebenfalls maximal zweimal, bevorzugt maximal einmal mit den genannten Gruppen substituiert sein können und
- M¹, M²: unabhängig voneinander, gleich oder verschieden, H, C₁-C₂₀-Alkyl
sind.

Weiterhin betrifft die Erfindung Verfahren zur Stabilisierung von unbelebtem organischen Material, insbesondere von Kunststoffen oder Lacken mit Hilfe von sterisch gehinderten Aminen, enthaltend eine oder mehrere Gruppen der allgemeinen Formel (I). Ein weiterer Gegenstand der Erfindung sind ausgewählte sterisch gehinderte Amine, enthaltend eine oder mehrere Gruppen der allgemeinen Formel (I). Ein weiterer Gegenstand der Erfindung sind Materialien, welche ausgewählte sterisch gehinderte Amine, enthaltend eine oder mehrere Gruppen der allgemeinen Formel (I), enthalten.

Die Stabilisierung der unbelebten organischen Materialien erfolgt bevorzugt durch den Schutz dieser Materialien vor negativen Auswirkungen von elektomagnetischer Strahlung, insbesondere Licht, Wärme oder Radikalen, insbesondere Sauerstoff.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt bzw. ganz bevorzugt sind die Ausführungsformen der vorliegenden Erfindung in denen alle Merkmale die bevorzugten bzw. ganz bevorzugten Bedeutungen haben.

EP 0 309 402 A1 beschreibt gehinderte Amine basierend auf 2,2,6,6-tetraalkylierten Stickstoff-enthaltenden heterocyclischen Gruppen, wobei das gehinderte Ring-Stickstoffatom durch OR-Gruppen substituiert ist und die 4-Position des Rings mit einer Vielzahl verschiedener Gruppen substituiert sein kann. Diese gehinderten Amine finden gemäß der EP 0 309 402 A1 Verwendung als Lichtstabilisatoren in unterschiedlichen Substraten, insbesondere thermoplastischen Kunststoffen.

US 5,004,770 beschreibt ebenfalls gehinderte Amine basierend auf 2,2,6,6-tetraalkylierten Stickstoff-enthaltenden heterocyclischen Gruppen, wobei das gehinderte Ring-Stickstoffatom durch OH oder OR-Gruppen substituiert ist und die 4-Position des Rings mit einer Vielzahl verschiedener Gruppen substituiert sein kann. Diese gehinderten Amine finden gemäß der US 5,004,770 Verwendung als Stabilisatoren gegen den Einfluß von Licht, Wärme oder Sauerstoff in unterschiedlichen Substraten, die keine Polyolefine sind.

US 5,096,950 beschreibt die Verwendung sterisch gehinderter Amine basierend auf 2,2,6,6-tetraalkylierten Stickstoff-enthaltenden heterocyclischen Gruppen, wobei das gehinderte Ring-Stickstoffatom durch OR-Gruppen substituiert ist und die 4-Position des Rings mit einer Vielzahl verschiedener Gruppen substituiert sein kann als Stabilisatoren gegen den Einfluß von Licht, Wärme oder Sauerstoff in Polyolefinen.

DE 101 60 602 A1 betrifft thermoplastisches organische Polymere, insbesondere Polyolefine, die gegen die schädlichen Einflüsse von Licht, Sauerstoff und. Wärme stabilisiert sind und ein bestimmtes flammhemmendes Mittel enthalten, das ein sterisch gehindertes Amin der Hydrocarbyloxyamin- oder Hydroxyhydrocarbyloxyamin-Klasse enthält. Ferner beschreibt die DE 101 60 602 A1 die Verwendung dieser flammhemmenden Mittel und der sterisch gehinderten Amine, um thermoplastische organisches Polymere gegen Licht zu stabilisieren und flammhemmend auszurüsten.

Lichtstabilisatoren auf Basis gehinderter Amine, die an dem gehinderten Stickstoffatom durch verschiedenartige OR-Gruppen substituiert sind, sind daher aus dem Stand der Technik bekannt.

Unbefriedigend ist oft die mangelhafte Verträglichkeit der gehinderten Amine mit Polyolefinen und anderen Kunststoffen sowie die Unverträglichkeit mit Säuren oder mit Materialien, welche bei Einwirkung von Licht und/oder Wärme Säuren bilden, die Dauer der Schutzwirkung, die Eigenfarbe der Substanzen und die thermische Zersetzung der Stabilisatoren beim Einarbeiten in Polymere bei erhöhter Temperatur.

Der Erfindung lag daher die Aufgabe zugrunde, weitere Stabilisatoren auf der Basis von gehinderten Aminen zur Verfügung zu stellen, welche eine weitere Verbesserung in der Dauer der Stabilisierung von unbelebtem organischem Material mit sich bringen, darüber hinaus aber auch gute Verträglichkeit mit den zu stabilisierenden Materialien, etwa Polyolefinen und anderen Kunststoffen, geringe Eigenfarbe und Beständigkeit bei der Einarbeitung in das zu stabilisierende Material, aufweisen.

Diese und andere Aufgaben werden, wie aus dem Offenbarungsgehalt der vorliegenden Erfindung ersichtlich, durch die verschiedenen Ausführungsformen der erfindungsgemäßen Verwendung der oben beschriebenen sterisch gehinderte Amine, enthaltend eine oder mehrere Gruppen der allgemeinen Formel (I) gelöst.

Selbstverständlich lassen sich im Rahmen der erfindungsgemäßen Verwendung auch Gemische von sterisch gehinderten Aminen, enthaltend eine oder mehrere Gruppen der allgemeinen Formel (I), einsetzen.

Im Rahmen der erfindungsgemäßen Verwendungen zeigen die sterisch gehinderte Amine, enthaltend eine oder mehrere Gruppen der allgemeinen Formel (I) keine Eigenfarbe, sind gut verträglich mit den verschiedensten unbelebten organischen Materialien, insbesondere organischen Polymeren, zeigen einen niedrigen Dampfdruck und damit geringe Flüchtigkeit, weisen eine hohe Migrationsstabilität auf, sind stabil gegen thermische Zersetzung und Säureeinwirkung und besitzen eine gegenüber den Verbindungen aus dem Stand der Technik verbesserte Stabilisierungsdauer.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Halogen steht für Fluor, Chlor, Brom, oder lod, vorzugsweise für Fluor, Chlor oder Brom, besonders bevorzugt für Fluor oder Chlor.

Im Einzelnen haben die für die verschiedenen Substituenten angegebenen Sammelbegriffe folgende Bedeutung:
C₁-C₂₀-Alkyl: geradkettige oder verzweigte Kohlenwasserstoffreste mit bis zu 20 Kohlenstoffatomen, beispielsweise C₁-C₁₀-Alkyl oder C₁₁-C₂₀-Alkyl, bevorzugt C₁-C₁₀-Alkyl beispielsweise C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes mehrcyclisches System wie z. B. Norbornyl oder Norbenyl. Besonders bevorzugt C₅-C₆-Cycloalkyl.

C₂-C₂₀-Alkenyl: ungesättigte, geradkettige oder verzweigte Kohlenwasserstoffreste mit 2 bis 20 Kohlenstoffatomen und mindestens einer Doppelbindung, bevorzugt einer Doppelbindung, in einer beliebigen Position, beispielsweise C₂-C₁₀-Alkenyl oder C₁₁-C₂₀-Alkenyl, bevorzugt C₂-C₁₀-Alkenyl wie C₂-C₄-Alkenyl, wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, oder C₅-C₆-Alkenyl, wie 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-bu-tenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl, sowie C₇-C₁₀-Alkenyl, wie die Isomere von Heptenyl, Octenyl, Nonenyl oder Decenyl.

C₁-C₂₀-Alkylen: geradkettige oder verzweigte Kohlenwasserstoffreste mit zwei freien Valenzen mit 1 bis 20 Kohlenstoffatomen, beispielsweise C₁-C₁₀-Alkylen oder C₁₁-C₂₀-Alkylen, bevorzugt C₁-C₁₀-Alkylen, insbesondere Methylen, Dimethylen, Trimethylen, Tetramethylen, Pentamethylen oder Hexamethylen.

C₂-C₂₀-Alkenylen: C₂-C₂₀-Alkenyl wie oben beschrieben mit zwei freien Valenzen.

C₃-C₁₂-Alkynoyl: Eine geradkettige oder verzweigte Alkinylgruppe mit 3 bis 20 Kohlenstoffatomen, welche über eine Carbonylgruppe (-CO-) angebunden ist und mindestens eine C-C-Dreifachbindung enthält. Bevorzugt ist C₃-C₁₀-Alkynoyl, z.B. Propinoyl, 2-Butinoyl, 3-Butinoyl.

C₂-C₂₀-Diacyl: Eine geradkettige oder verzweigte Alkylgruppe mit 2 bis 20 Kohlenstoffatomen, die über zwei Carbonylgruppen (-CO-) angebunden ist. Bevorzugt ist C₂-C₁₀-Diacyl, z.B. Oxalyl, Malonyl, Succinyl, Glutaryl, Sebacoyl.

C₄-C₂₀-Triacyl: Eine verzweigte Alkylgruppe mit 4 bis 20 Kohlenstoffatomen, die über drei Carbonylgruppen (-CO-) angebunden ist. Bevorzugt ist C₄-C₁₀-Triacyl, z.B. 1,2,3-Propantricarbonyl.

C₅-C₂₀-Tetracyl: Eine verzweigte Alkylgruppe mit 5 bis 20 Kohlenstoffatomen, die über vier Carbonylgruppen (-CO-) angebunden ist. Bevorzugt ist C₅-C₁₀-Tetracyl, z.B. Methantetracarbonyl oder 1,2,3,4-Butantetracarbonyl.

C₁-C₂₁-Alkanoyl: ein Wasserstoffatom oder eine geradkettige oder verzweigte Alkylgruppe mit 1 bis 20 Kohlenstoffatomen (wie vorstehend genannt), welche über eine Carbonylgruppe (-CO-) angebunden ist, bevorzugt C₁-C₁₃-Alkanoyl wie beispielsweise Formyl, Acetyl, n-oder iso-Propionyl, n-, iso-, sec- oder tert.-Butanoyl, n-iso-, sec- oder tert.-Pentanoyl, n- oder iso-Nonanoyl, n-Dodecanoyl.

Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.

Arylen: Aryl mit zwei freien Valenzen, wobei bezogen auf das zugehörige Aryl ein weiteres Wasserstoffatom am aromatischen Ringsystem fehlt, beispielsweise Phenylen, Naphthylen.

Arylalkyl ist ein ein- bis dreikerniges aromatisches Ringsystem (wie vorstehend genannt), welches über eine C₁-C₂₀-Alkylengruppe angebunden ist, bevorzugt ein ein- bis zweikerniges, besonders bevorzugt ein einkerniges aromatisches Ringsystem.

Heterocyclen: fünf- bis zwölfgliedrige, bevorzugt fünf- bis neungliedrige, besonders bevorzugt fünf- bis sechsgliedrige, Sauerstoff-, Stickstoff- und/oder Schwefelatomenthaltende, gegebenenfalls mehrere Ringe aufweisende und/oder substituierte Ringsysteme wie Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) an gebunden ist, beispielsweise C₁-C₁₀-Alkoxy oder C₁₁-C₂₀-Alkoxy, bevorzugt C₁-C₁₀-Alkyloxy, insbesondere bevorzugt C₁-C₃-Alkoxy, wie beispielweise Methoxy, Ethoxy, Propoxy.

C₄-C₂₂-Alkoxyalkyl: Eine geradkettige oder verzweigte Alkylkette mit 4 bis 22 Kohlenstoffatomen, die durch ein Sauerstoffatom unterbrochen ist. Bevorzugt sind solche Ketten, die sowohl vor als auch hinter dem Sauerstoffatom mindestens zwei Kohlenstoffatome enthalten.

Heteroatome sind bevorzugt Sauerstoff, Stickstoff, Schwefel oder Phosphor. Gegebenenfalls sind die freien Valenzen der Heteroatome durch Wasserstoffatome abgesättigt.

Bevorzugt werden im Rahmen der erfindungsgemäßen Verwendung sterisch gehinderte Amine, enthaltend eine oder mehrere Gruppen der allgemeinen Formel (I) mit R¹, R² unabhängig voneinander, gleich oder verschieden, H, C₁-C₂₀-Alkyl oder Aryl eingesetzt, besonders bevorzugt sind R¹, R² unabhängig voneinander, gleich oder verschieden, H, C₁-C₂₀-Alkyl und insbesondere sind R¹ und R² beide gleich H.

Weiterhin bevorzugt werden im Rahmen der erfindungsgemäßen Verwendungen sterisch gehinderten Amine Verbindungen der allgemeinen Formeln (I-a) bis (I-t) eingesetzt:

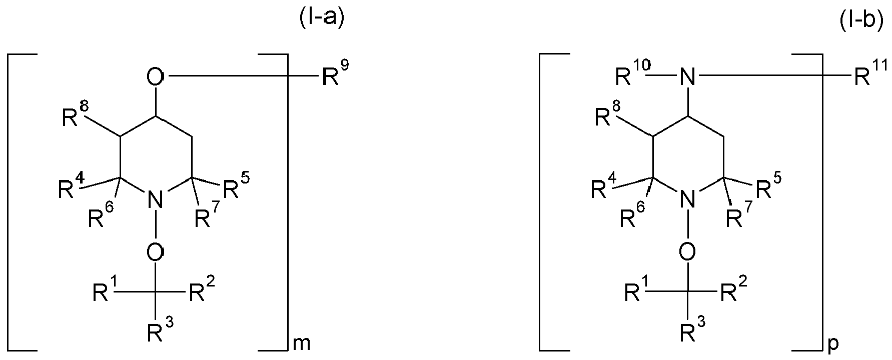

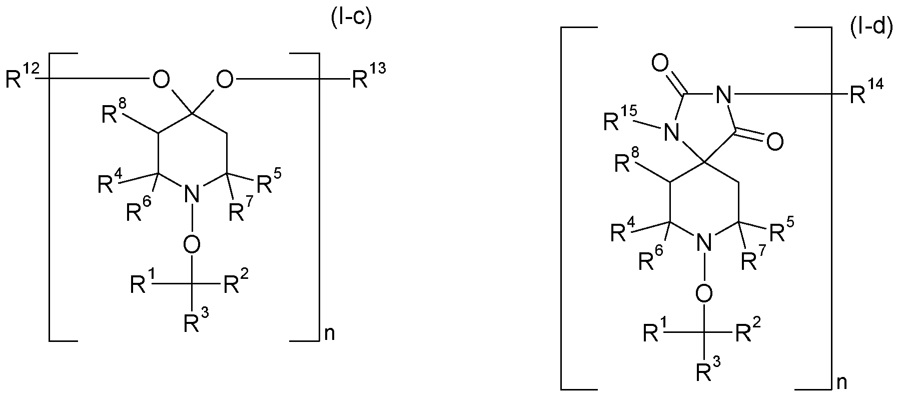

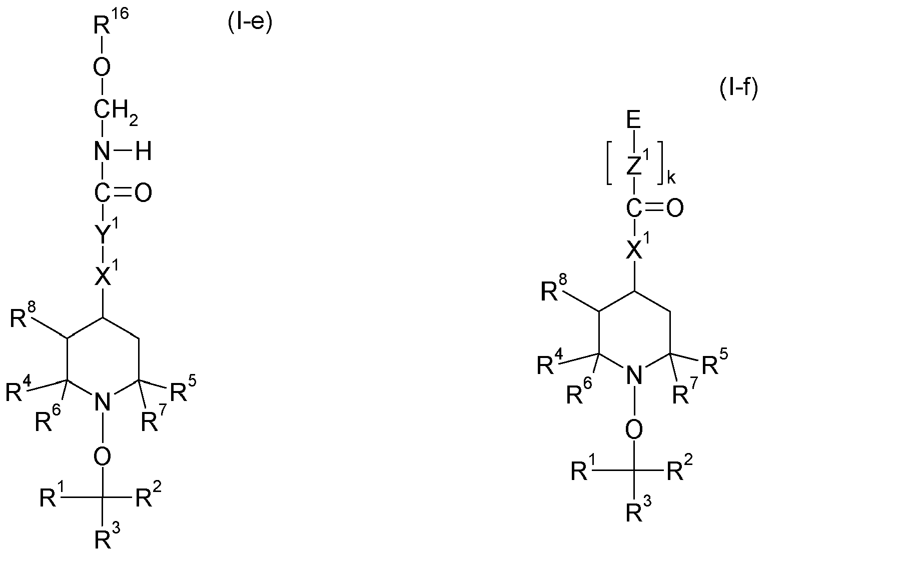

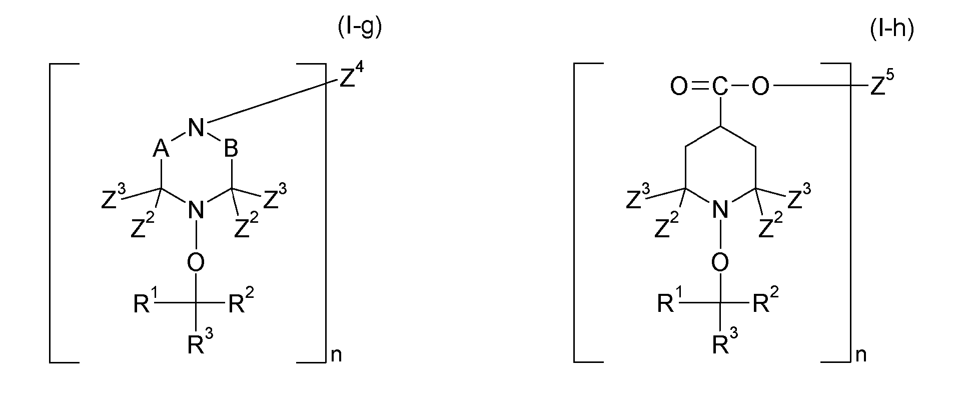

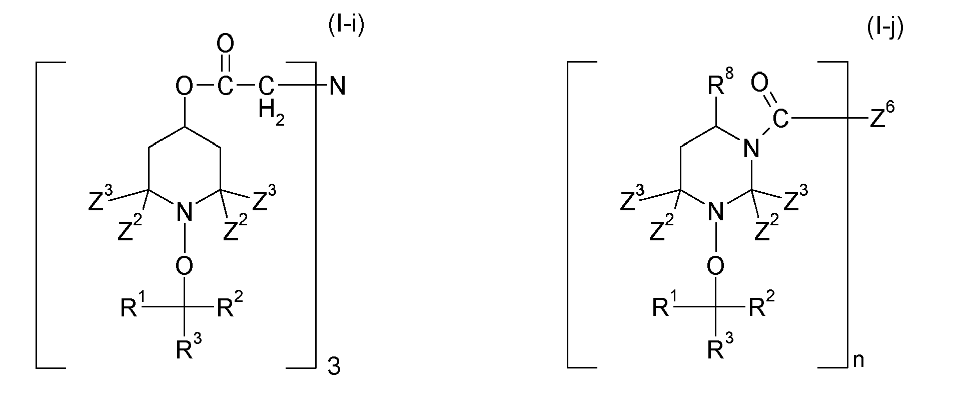

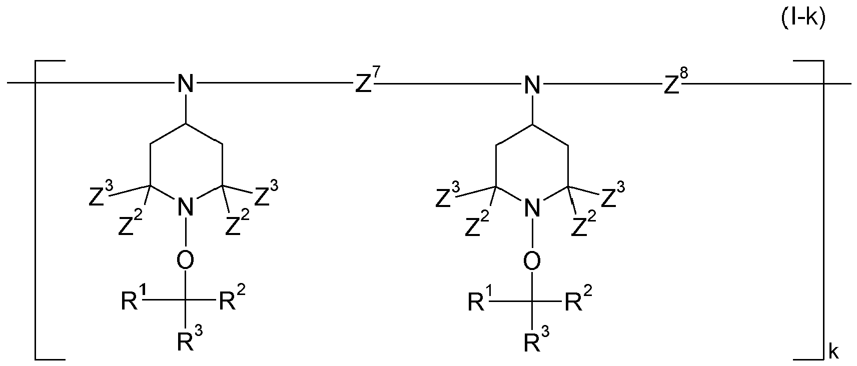

Die Endgruppen der Verbindung (I-k) sind hierbei beliebig und von untergeordneter Bedeutung. Beispielsweise kommen als Endgruppen H, OH, C₁-C₂₀-Alkyl, Aryl, C₁-C₂₀-Alkoxy, oder C₂-C₂₀-Alkenyl in Frage.

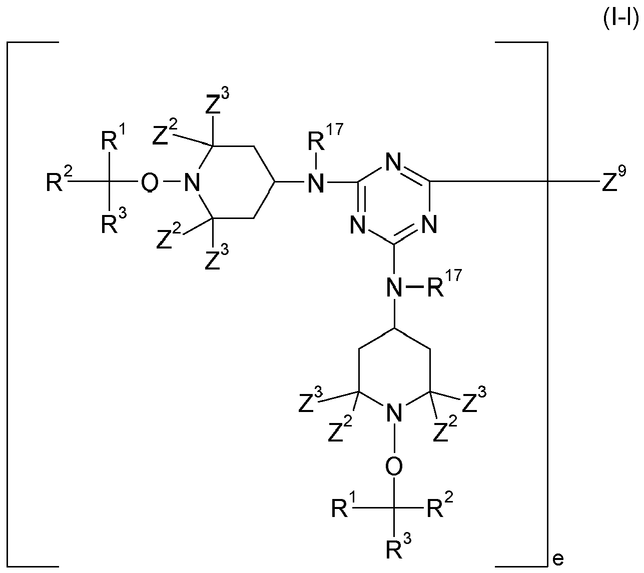

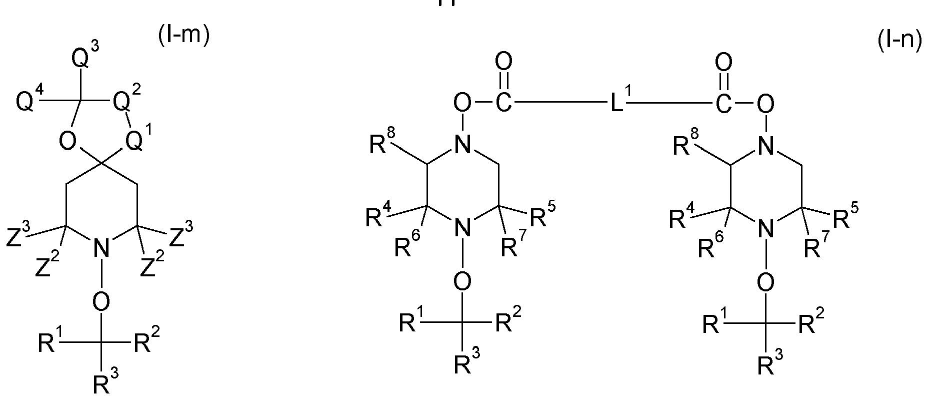

### (1-o) - Kondensationsprodukt von (I-m) mit Epichlorhydrin (symbolisch):

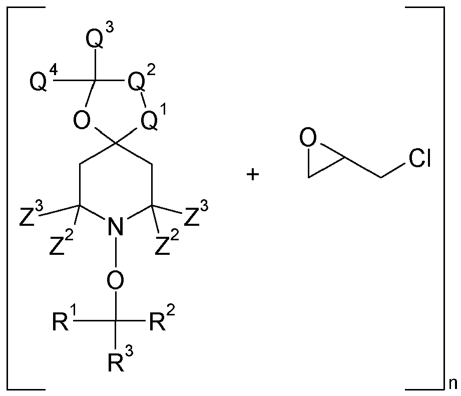

bevorzugt: 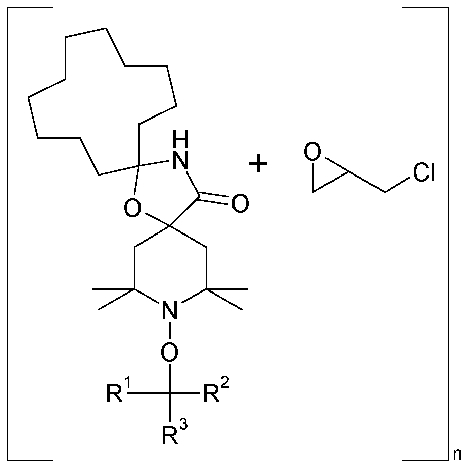 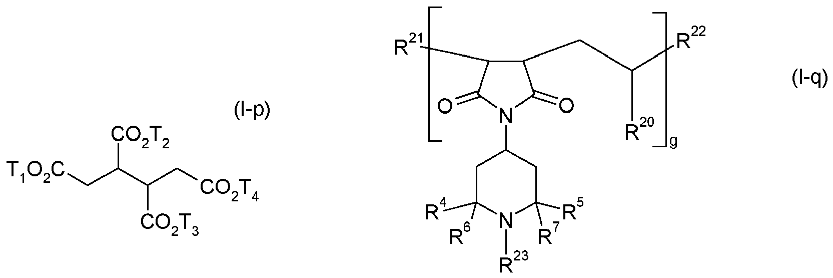 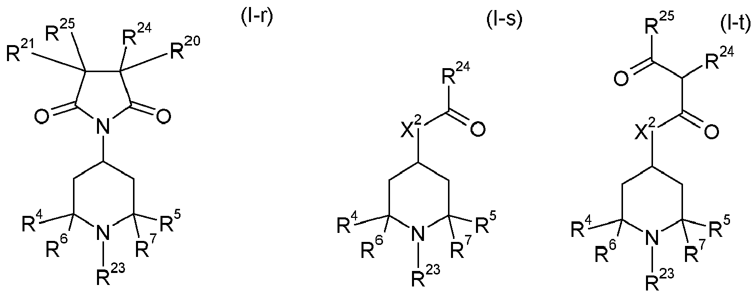 sind, wobei R¹ bis R⁷, wie in Anspruch 1 definiert sind, mit
- R⁸: H, CH₃,
- R¹⁰: H, C₁-C₂₀-Alkyl, C₃-C₁₅-Cycloalkyl, C₇-C₉-Aralkyl, C₁-C₂₁-Alkanoyl, Benzoyl,
- R¹⁵: H, C₁-C₂₀-Alkyl, Allyl, Benzyl, Glycidyl, C₁-C₂₀-Alkoxyalkyl,
- X¹: -N(R¹⁸)-, -O-
- R¹⁸: H, C₁-C₂₀-Alkyl, C₃-C₁₅-Cycloalkyl, C₇-C₁₂-Aralkyl, Cyanoethyl, Aryl, -CH₂-CH(R¹⁹)-OH, eine Gruppe der allgemeinen Formel (II), (III), (IV) oder (V),

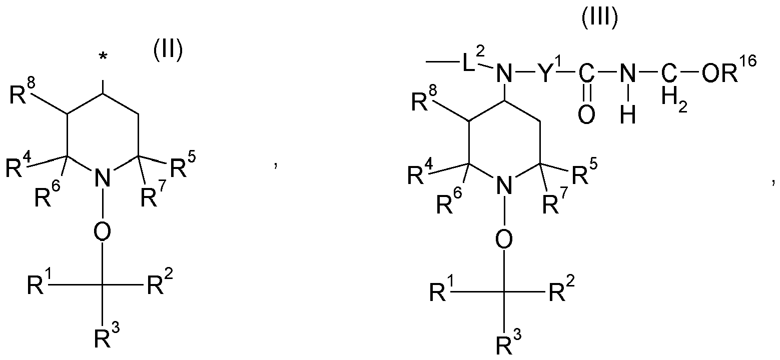

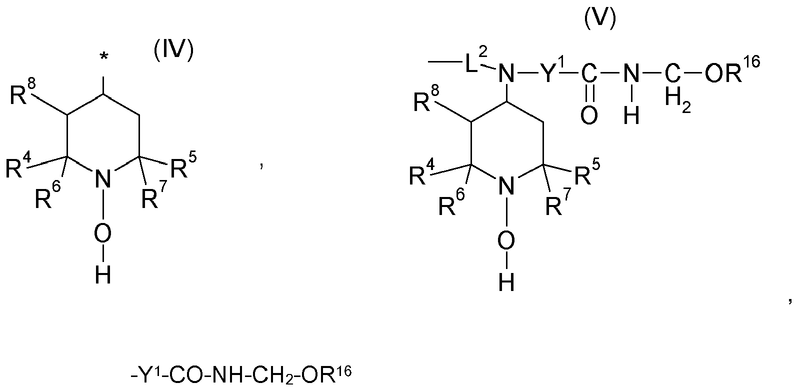

- R¹⁹: H, CH₃, Phenyl,
- R¹⁶: H, C₁-C₂₀-Alkyl, C₂-C₂₀-Alkenyl, Aryl, Hetaryl
- Y¹: C₁-C₃-Alkylen, -CH₂-CH(R¹⁹)-O-, -(CH₂)₃-NH-, Einfachbindung,
- L²: C₂-C₆-Alkylen, Arylen,
- A, B: unabhängig voneinander, gleich oder verschieden, Methylen, Carbonyl,
- m: 1,2,3,4,
und
falls m = 1 ist,
- R⁹: H, C₁-C₂₀-Alkyl, C₂-C₂₀-Alkenyl, Aryl, C₇-C₂₀-Aralkyl, Glycidyl, C₂-C₂₂-Alkanoyl, eine Gruppe der allgemeinen Formel (VI) oder (VII) 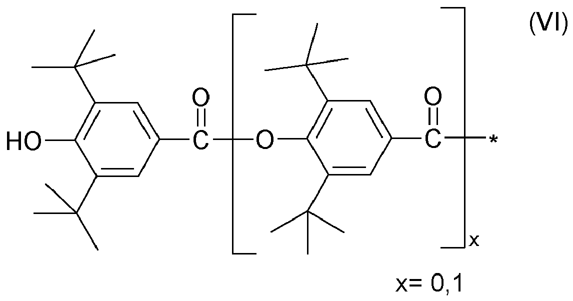 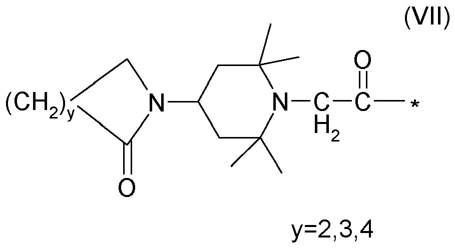
und
falls m = 2 ist,
- R⁹: C₁-C₂₀-Alkylen, C₂-C₂₀-Alkenylen, Xylylen, C₂-C₂₀-Di-acyl, eine Gruppe der allgemeinen Formel (VIII) oder (IX) 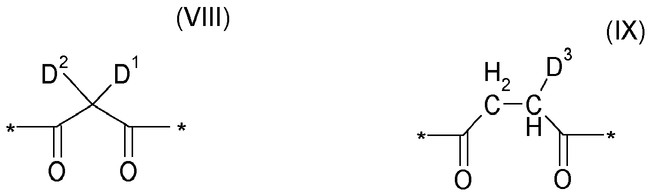
- D¹, D²: unabhängig voneinander, gleich oder verschieden, H, C₁-C₂₀-Alkyl, Phenyl, Benzyl, 3,5-Di-t-butyl-4-hydroxy-benzyl oder D¹ und D² zusammen C₃-C₆-Alkylen,
- D³: C₁-C₂₀-Alkyl, C₂-C₂₀-Alkenyl,
und falls m = 3 ist,
- R⁹: C₄-C₂₀-Tri-acyl,
und
falls m = 4 ist,
- R⁹: C₅-C₂₀-Tetra-acyl
- p: 1,2,3,
und falls p = 1 ist,
- R¹¹: H, C₁-C₂₀-Alkyl, C₃-C₁₅-Cycloalkyl, C₂-C₂₀-Alkenyl, C₁-C₂₁-Alkanoyl, Carbonyl, Carbamid, Aryl, C₇-C₁₀-Aralkyl, Glycidyl, -CH₂-CH(OH)-H, -CH₂-CH(OH)-CH₃, -CH₂-CH(OH)-Phenyl, eine Gruppe der allgemeinen Formel (II) oder (IV), oder R¹⁰ und R¹¹ bilden gemeinsam ein C₄-C₆-Alkylen oder 1-Oxoalkylen, oder ein C₂-C₂₀-Di-alkanoyl
und falls p = 2 ist,
- R¹¹: C₁-C₁₂-Alkylen, Xylylen, --CH₂-CH(OH)-CH₂-, O-(C₂-C₁₀-Alkylen)-O-CH₂-CH(OH)-CH₂-, Arylen, C₆-C₁₂-C_{YC}loalkylen, unter der Maßgabe, dass R¹⁰ kein Alkynoyl, Alkenoyl oder Benzoyl ist, kann R¹¹ auch ein C₂-C₂₀-Di-acyl sein, oder-CO-, eine Gruppe der allgemeinen Formel (X) 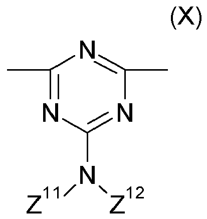 mit Z¹¹, Z¹² unabhängig voneinander, H, C₁-C₂₀-Alkyl, eine Gruppe der allgemeinen Formel (II), oder Z¹¹ und Z¹² bilden gemeinsam ein C₄-C₆-Alkylen oder 3-Oxapentamethylen,
und falls p = 3 ist,
- R¹¹: 2,4,6-Triazintriyl,
- n: 1,2
und falls n = 1 ist,
- R¹²: C₁-C₂₀-Alkyl, C₂-C₂₀-Alkenyl, C₇-C₁₂-Aralkyl,
- R¹³: H, C₁-C₂₀-Alkyl, C₂-C₂₀-Alkenyl, C₇-C₁₂-Aralkyl, oder R¹³ bildet gemeinsam mit R¹² ein C₂-C₈-Alkylen, C₂-C₈-Hydroxyalkylen, C₄-C₂₂-Acyloxyalkylen,
- R¹⁴: H, C₁-C₂₀-Alkyl, C₃-C₅-Alkenyl, C₇-C₉-Aralkyl, C₃-C₁₅-Cycloalkyl, C₂-C₄-Hydroxyalkyl, C₂-C₆-Alkoxyalkyl, Aryl, Glycidyl, -(CH₂)ₜ-COO-Q, -(CH₂)ₜ-O-CO-Q, mit t = 1, 2 und Q = C₁-C₄-Alkyl, Phenyl,
und falls n = 2 ist,
- R¹², R¹³: (-CH₂)₂C(CH₂-)₂,
- R¹⁴: C₂-C₂₀-Alkylen, Arylen, -CH₂CH(OH)-CH₂-O-X-CH₂-CH(OH)-CH₂-, mit X = C₂-C₁₀-Alkylen, Arylen, C₆-C₁₂-Cycloalkylen, oder -CH₂CH(OZ')-CH₂-(O-CH₂-CH(OZ')CH₂)₂, mit Z' = H, C₁-C₂₀-Alkyl, Allyl, Benzyl, C₃-C₁₂-Alkynoyl, Benzoyl,
- Z¹: Ethylen, 1,2-Propylen, Wiederholungseinheit eines Polymers oder Copolymers abgeleitet von einem oder mehreren Alpha-Olefinen,
- k: eine ganze Zahl aus dem Bereich von 2 bis 100,
- E: H, C₁-C₂₀-Alkyl, Aryl, C₁-C₂₀-Alkoxy, C₂-C₂₀-Alkenyl,
- Z⁴: die gleich Bedeutung wie R¹¹ wenn p = 1 oder 2,
- Z²: Methyl,
- Z³: Methyl, Ethyl, oder Z³ und Z² bilden gemeinsam eine Tetramethylen oder Pentamethylengruppe,
- Z⁵: die gleiche Bedeutung wie R¹⁴,
- Z⁷, Z⁸: unabhängig voneinander, gleich oder verschieden, C₂-C₁₂-Alkylen, oder Z⁷ ist eine Gruppe der allgemeinen Formel (VIII),
- e: 2,3,4,
- Z⁹: -N(R¹³)-(CH₂)_{d}-N(R¹³)-, oder eine Gruppe der allgemeinen Formel (XI) 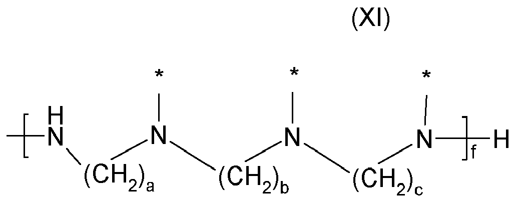 mit a, b, c unabhängig voneinander, gleich oder verschieden 2 oder 3 und d eine ganze Zahl aus dem Bereich von 2 bis 10 und f = 0 oder 1,
- R¹⁷: die gleiche Bedeutung wie R¹⁰,
- Z⁶: die gleich Bedeutung wie R¹¹,
- Q¹, Q²: verschieden voneinander, jeweils -CO-, N(Q⁵)-
- Q⁵: H, C₁-C₂₀-Alkyl, C₄-C₂₀-Alkoxycarbonyl,
- Q³: H, C1-C₂₀-Alkyl, Phenyl, Naphthyl,
- Q⁴: H, C1-C₂₀-Alkyl, Phenyl, Naphthyl, C₇-C₁₂-Phenylalkyl, oder Q4 und Q3 bilden gemeinsam ein C₄-C₁₇-Alkylen,
- L1: Einfachbindung, C₁-C₁₂-Alkylen, Phenylen, -NH-(C₁-C₁₂-Alkylen)-NH-
- T¹,T²,T³,T⁴: unabhängig voneinander, gleich oder verschieden C2-C20-Alkyl, oder ein Strukturelement der allgemeinen Formel (II), unter der Maßgabe, dass mindestens einer der Substituenten T¹, T², T³ oder T⁴ einem Strukturelement der allgemeinen Formel (II) entspricht
- g: eine ganze Zahl aus dem Bereich von 2 bis 1000,
- R²⁰: C₁-C₃₀-Alkyl, Aryl, Gemisch verschiedener C₁-C₃₀-Alkyl,
- R²¹, R²²: unabhängig voneinander, gleich oder verschieden, H, C₁-C₂₀-Alkyl, Aryl, C₁-C₂₀-Alkoxy, C₂-C₂₀-Alkenyl,
- R²³: H, O, OH, C₁-C₂₀-Alkyl, -OC(R¹R²R³), unter der Maßgabe, dass mindestens einer der Substituenten R²³ -OC(R¹R²R³) ist,
- R²⁴, R²⁵: unabhängig voneinander, gleich oder verschieden, C₁-C₂₄-Alkyl, bevorzugt C₁₀-C₂₄-Alkyl , besonders bevorzugt C₁₄-C₂₂-Alkyl, ganz besonders bevorzugt C₁₈-C₂₂-Alkyl,
- X²: O, NH
und
wobei R⁹, R¹¹, Q³, Q⁴, jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch C₁-C₂₀-Alkyl, C₁-C₂₀-Alkoxy, Aryl, Heterocyclus, Heteroatome oder Halogen substituiert sein können, wobei diese ebenfalls maximal zweimal, bevorzugt maximal einmal mit den genannten Gruppen substituiert sein können.

Selbstverständlich lassen sich im Rahmen der erfindungsgemäßen Verwendung auch Gemische von sterisch gehinderten Aminen der Formeln (I-a) bis (I-t) einsetzen.

Besonders bevorzugt werden im Rahmen der erfindungsgemäßen Verwendungen sterisch gehinderten Amine Verbindungen der allgemeinen Formeln I-a, I-b, I-k, I-I, I-m, 1-o, I-p und I-q eingesetzt. Ganz besonders bevorzugt sind sterisch gehinderten Amine Verbindungen der allgemeinen Formeln I-b und I-q .

In einer weiteren bevorzugten Ausführungsform der erfindungsgemäßen Verwendung haben die Substituenten R⁴, R⁵, R⁶, R⁷ in den Formeln (I) sowie (I-a) bis (I-t) alle die Bedeutung einer Methyl-Gruppe.

In einer weiteren bevorzugten Ausführungsform der erfindungsgemäßen Verwendung ist der Substituent R⁸ in den Formeln (I-a) bis (I-t) ein Wasserstoffatom.

In einer weiteren besonders bevorzugten Ausführungsform der erfindungsgemäßen Verwendung haben die Substituenten R⁴, R⁵, R⁶, R⁷ in den Formeln (I-a) bis (I-t) alle die Bedeutung einer Methyl-Gruppe und der Substituent R⁸ ist ein Wasserstoffatom.

In einer weiteren bevorzugten Ausführungsform der erfindungsgemäßen Verwendung entspricht das sterisch gehinderte Amin der Formel (I-a) und R⁴, R⁵, R⁶, R⁷ sind alle Methyl, R⁸ ist Wasserstoff, m = 1 und R⁹ ist ein Gemisch von C₁₂-C₂₁-Alkanoylen, insbesondere ein Gemisch von C₁₂-C₂₁-Alkanoylen bestehend zum größten Teil aus C₁₇-C₁₉-Alkanoylen.

In einer weiteren bevorzugten Ausführungsform der erfindungsgemäßen Verwendung entspricht das sterisch gehinderte Amin der Formel (I-a) und R⁴, R⁵, R⁶, R⁷ sind alle Methyl, R⁸ ist Wasserstoff, m = 2 und R⁹ ist C₁₀-Diacyl.

In einer weiteren bevorzugten Ausführungsform der erfindungsgemäßen Verwendung entspricht das sterisch gehinderte Amin der Formel (I-b) und R⁴, R⁵, R⁶, R⁷ sind alle Methyl, R⁸ ist Wasserstoff, R¹⁰ ist Formyl, p = 2 und R¹¹ ist Hexamethylen.

In einer weiteren bevorzugten Ausführungsform der erfindungsgemäßen Verwendung entspricht das sterisch gehinderte Amin der Formel (I-k) und Z², Z³ sind beide Methyl, Z⁷ ist eine Gruppe der allgemeinen Formel (X), Z¹¹ ist Wasserstoff, Z¹² ist tert-octyl und Z⁸ ist Hexamethylen. Die Endgruppen der Kette ergeben sich durch die Herstellbedingungen. Zum Beispiel sind die Ketten am Ende mit H oder OH substituiert.

In einer weiteren bevorzugten Ausführungsform der erfindungsgemäßen Verwendung entspricht das sterisch gehinderte Amin der Formel (I-k) und Z², Z³ sind beide Methyl, Z⁷ ist eine Gruppe der allgemeinen Formel (X), Z¹¹ ist n- butyl, Z¹² ist eine Gruppe der allgemeinen Formel (II), und Z⁸ ist Hexamethylen und R⁴, R⁵, R⁶, R⁷ sind alle Methyl. Die Endgruppen der Kette ergeben sich durch die Herstellbedingungen. Zum Beispiel sind die Ketten am Ende mit H oder OH substituiert

In einer weiteren bevorzugten Ausführungsform der erfindungsgemäßen Verwendung entspricht das sterisch gehinderte Amin der Formel (I-I) und Z², Z³ sind beide Methyl, R¹⁷ ist n- butyl, e = 4, Z⁹ ist eine Gruppe der allgemeinen Formel (XI), und a = 3, c=3, b =2,f=1.

In einer weiteren bevorzugten Ausführungsform der erfindungsgemäßen Verwendung entspricht das sterisch gehinderte Amin der Formel (I-m) und Z², Z³ sind beide Methyl, Q³ und Q⁴ bilden zusammen ein C₁₁-Alkylen und Q¹ ist C=O und Q² ist N-H.

In einer weiteren bevorzugten Ausführungsform der erfindungsgemäßen Verwendung entspricht das sterisch gehinderte Amin der Formel (I-o) und Z², Z³ sind beide Methyl, Q³ und Q⁴ bilden zusammen ein C₁₁-Alkylen und Q¹ ist C=O und Q² ist N-H.

In einer weiteren bevorzugten Ausführungsform der erfindungsgemäßen Verwendung entspricht das sterisch gehinderte Amin der Formel (I-p) und T¹, T², T³, T⁴ sind alle eine Gruppe der allgemeinen Formel (II), R⁴, R⁵, R⁶, R⁷ sind alle Methyl und R⁸ ist Wasserstoff.

In einer weiteren bevorzugten Ausführungsform der erfindungsgemäßen Verwendung entspricht das sterisch gehinderte Amin der Formel (I-p) wobei T¹, T², T³, T⁴ C₁₃-Alkyl, oder ein Gemisch von C₁₃-Alkylen, oder eine Gruppe der allgemeinen Formel (II), mit R⁴, R⁵, R⁶, R⁷ alle Methyl und R⁸ Wasserstoff, sind.

In einer weiteren bevorzugten Ausführungsform der erfindungsgemäßen Verwendung entspricht das sterisch gehinderte Amin der Formel (I-q) wobei R²⁰ ein Gemisch aus C₁₈-C₂₂-Alkylgruppen, insbesondere ein Gemisch aus C₁₈-Alkyl, C₂₀-Alkyl und C₂₂-Alkyl ist. Besonders bevorzugt sind hierbei sterische Amine der Formel (I-q), für die gilt, dass R²⁰ eine Mischung aus C₁₈-C₂₂-Alkylgruppen, insbesondere ein Gemisch aus C₁₈-Alkyl, C₂₀-Alkyl und C₂₂-Alkyl ist und die Mehrzahl (mehr als 50%) der Substituenten R²³ einer Gruppe -OCR¹R²R³ entsprechen.

Sterisch gehinderte Amine, enthaltend eine oder mehrere Gruppen der allgemeinen Formel (I) und sterisch gehinderte Amine der Formel (I-a) bis (I-t) können gemäß dem Fachmann bekannten Verfahren hergestellt werden. Diese Verbindungen werden im Allgemeinen durch Oxidation der entsprechenden N-H Derivate mit einer geeigneten Peroxiverbindung, beispielsweise Wasserstoffperoxid oder Tert-butyl-peroxid in der Gegenwart eines Metallcarbonyl oder Metalloxidkatalysators und anschließender Reduktion der Oxylverbindung zum entsprechenden N-OH Derivat, insbesondere durch katalytische Hydrogenierung, hergestellt. Die N-OH Derivate können dann mit Hilfe von Natriumhydrid und halogenierten Kohlenwasserstoffen oder deren Derivaten, beispielsweise Benzylbromid, alkyiert werden (Entezami et al., Polym. Adv. Technol. 2007, 18, 306-312).

Alternativ kann auch eine Kopplung von Nitroxylradikalen mit Kohlenwasserstoffradikalen durchgeführt werden, wobei die Kohlenwasserstoffradikale, oder deren Derivate, durch eine thermische Zersetzung von Di-tert-butyl-peroxid in der Gegenwart der Kohlenwasserstoffe, beispielsweise in der Gegenwart von Toluol oder Ethylbenzol, erzeugt werden (Hawker et al., Macromolecules 1996, 29, 5245).

Ein weiteres Verfahren zur Herstellung sterisch gehinderter Amine, enthaltend eine oder mehrere Gruppen der allgemeinen Formel (I) und sterisch gehinderter Amine der Formel (I-a) bis (I-t) geht unmittelbar von den N-H Derivaten aus und umfasst die Oxidations- und Alkylierungsschritte in einem einzigen Verfahrensschritt ohne die Isolierung der N-OH oder Nitroxylradikalderivate (WO 2008/003602 A1).

Die N-H Derivate sind als Ausgangsprodukte häufig kommerziell erhältlich oder können mit Hilfe von dem Fachmann bekannten Methoden hergestellt werden, wie beispielweise in EP 0 316 582 A1, EP 0 316 582 A, WO 94/12544 beschrieben.

In einer bevorzugten Ausführungsform der erfindungsgemäßen Verwendung sind die stabilisierten unbelebten organischen Materialien Kunststoffe oder Lacke, insbesondere Kunststoffe. Ganz bevorzugt sind diese Kunststoffe Agrarfolien, insbesondere Gewächshausfolien, die bevorzugt Polyethylen enthalten. Weitere bevorzugte Kunststoffe sind PVC oder flammgeschützte Thermoplasten, insbesondere flammgeschütztes Polypropylen, ABS oder Polystyrol.

Als weitere Kunststoffe oder Polymere, die als unbelebte organische Materialen stabilisiert werden können, seien beispielsweise genannt:
Polymere von Mono- und Diolefinen, wie z.B. Polyethylen niedriger oder hoher Dichte, lineares Polybuten-1, Polyisopren, Polybutane sowie Copolymerisate von Mono- oder Diolefinen oder Mischungen der genannten Polymeren; Copolymerisate von Mono- oder Diolefinen mit anderen Vinylmonomeren wie z.B. Ethylen-Alkylacrylat-Copolymere,Ethylen-Alkylmethacrylat-Copolymere. EthylenVinylacetat-Copolymere oder Ethylen-Acrylsaure-Copolymere; Polystyrol; Copolymere von Styrol oder alpha-Methylstyrol mit Dienen oder Acrylderivaten, wie z.B. Styrol-Butadien, Styrol-Arcylnitril, Styrol-Ethylmethacrylat, Styrol-Butadien-Ethylacrylat, Styrol-Acrylnitril-Methacrylat; ABS-, MBS- oder ähnliche Polymere; Halogenhaltige Polymere, wie z.B. Polyvinylchlorid, Polyvinylfluorid, Polyvinylidenflourid sowie deren Copolymere; Polymere die sich von alpha,beta-ungesättigten Säuren und deren Derivaten ableiten, wie Polyacrylate, Polymethacrylate, Polyacrylamide und Polyacrylnitrile; Polymere, die sich von ungesättigten Alkoholen und Aminen bzw. von deren Acrylderivaten oder Acetalen ableiten, z.B. Polyvinylalkohol und Polyvinylacetat; Polyurethane, Polyamide, Polyharnstoff, Polyester, Polycarbonate, Polysulfone, Polyethersulfone und Polyetherketone.

Weiterhin können entsprechen der erfindungsgemäßen Verwendung, gegebenenfalls unter Zugabe ein oder mehrerer Antioxidatien, erfindungsgemäß Lacke stabilisiert werden, vor allem solche Lacke bzw. Lackierungen, welche in hohem Maße der Einwirkung von Umwelteinflüssen, wie Sonnenlicht und Hitze ausgesetzt sind. Dies sind z.B. Lacke für Aussenanstriche, Industrie- oder Fahrzeuglackierungen. Weiter sind dies Lacke für Einbrennlackierungen, vor allem im Automobilsektor. Die sterisch gehinderten Amine können in fester oder gelöster Form dem Lack zugesetzt werden. Ihre in der Regel gute Löslichkeit in Lacksystemen ist dabei von besonderem Vorteil.

Bevorzugt werden die sterisch gehinderten Amine, enthaltend eine oder mehrere Gruppen der allgemeinen Formel (I), insbesondere sterisch gehinderte Amine der Formel (I-a) bis (I-t), zum Stabilisieren von Polyolefinen, insbesondere von Ethylen- oder Propylenpolymerisaten, von Polyurethanen sowie von Polyamiden verwendet.

Die sterisch gehinderten Amine, enthaltend eine oder mehrere Gruppen der allgemeinen Formel (I), insbesondere sterisch gehinderte Amine der Formel (I-a) bis (I-t), werden dem zu stabilisierenden unbelebten organischen Material, insbesondere den zu stabilisierenden Kunststoffen, in einer Menge von 0,01 bis 5 Gew.-%, vorzugsweise von 0,02 bis 1 Gew.-%, bezogen auf das unbelebte organische Material zugesetzt. Wird das unbelebte organische Material aus kleineren Molekülen aufgebaut, wie etwa bei der Herstellung von Kunststoffen aus den entsprechenden Monomeren, so kann die Zugabe vor, während oder nach der Synthese des organischen Materials geschehen.

Zur Vermischung der sterisch gehinderten Amine und gegebenenfalls weiterer unten aufgeführter Additive mit dem organischen Material, insbesondere den Kunststoffen und Lacken, können alle bekannten Vorrichtungen und Methoden zum Einmischen von Stabilisierungsmitteln oder anderen Zusätzen in organische Materialien, z.B. Polymere, angewandt werden.

Beispielsweise können die sterisch gehinderten Amine und gegebenenfalls andere Additive dem Polymeren leicht durch herkömmliche Techniken in einem beliebigen Stadium vor der Herstellung der daraus gebildeten Formkörper einverleibt werden. Beispielsweise können diese mit dem Polymeren in trockener Pulverform vermischt werden oder eine Suspension oder Emulsion des Stabilisators kann mit einer Lösung, Suspension oder Emulsion des Polymeren vermischt werden. Die sterisch gehinderten Amine können einzeln oder im Gemisch untereinander zugesetzt werden. Gegebenenfalls können die einzelnen Komponenten miteinander beim Schmelzen (Schmelzvermischen) vor der Einverleibung in das zu stabilisierende Material vermischt werden. Die sterisch gehinderten Amine sowie gegebenenfalls weitere Additive können ferner dem zu stabilisierenden Material in Form einer Vormischung zugesetzt werden, die diese Komponenten in einer Konzentration von beispielsweise etwa 2,5 bis etwa 25 Gew.-% enthält. Bei diesen Vorgängen kann das Polymere in Form von Pulvern, Granulaten, Lösungen, Suspensionen oder Latices eingesetzt werden. Das Vermischen kann vor oder während des Formgebungsvorgangs durchgeführt werden. Im Fall von Elastomeren können diese in Form von Latices stabilisiert werden.

Eine weitere Möglichkeit zur Einverleibung der erfindungsgemäßen Stabilisatoren in Polymere besteht in ihrer Zugabe vor, während oder direkt nach der Polymerisation der entsprechenden Monomeren. Im Fall der Zugabe vor oder während der Polymerisation können die erfindungsgemäßen Stabilisatoren auch als Kettenlängenregulatoren der Polymeren (Kettenterminatoren) wirken.

In einer weiteren bevorzugten Ausführungsform der erfindungsgemäßen Verwendung können neben den sterischen gehinderten Aminen zusätzlich weitere Additive, beispielsweise UV-Absorber, Antioxidantien, Costabilisatoren, Metalldesaktivatoren, Metallseifen, Weichmacher, antistatische Mittel, Gleitmittel, Trennmittel, Verarbeitungshilfsmittel, Antiblockmittel, Antibeschlagsmittel, Flammschutzmittel, Pigmente, Farbstoffe, IR-Strahlung regulierende Verbindungen, Schäumungsmittel, Nukleierungsmittel und Füllstoffe eingesetzt werden.

Das durch die sterisch gehinderte Amine, enthaltend eine oder mehrere Gruppen der allgemeinen Formel (I), insbesondere sterisch gehinderte Amine der Formel (I-a) bis (1-t), stabilisierte unbelebte organische Material, insbesondere Kunststoffe und Lacke, kann daher gegebenenfalls noch weitere Additive enthalten, z.B. UV-Absorber, Antioxidantien, Costabilisatoren, Metalldesaktivatoren, Metallseifen, Weichmacher, antistatische Mittel, , Gleitmittel, Trennmittel, Verarbeitungshilfsmittel, Antiblockmittel, Antibeschlagsmittel, Flammschutzmittel, Pigmente, Farbstoffe, IR-Strahlung regulierende Verbindungen, Schäumungsmittel, Nukleierungsmittel und Füllstoffe. Wahlweise finden beispielsweise wenigstens ein oder mehrere weitere Additive a) bis t) Verwendung:
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine,
h) Antioxidantien,
i) Nickelverbindungen,
j) sterisch gehinderte Amine, die nicht eine oder mehrere Gruppen der allgemeinen Formel (I) enthalten,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen,
s) basische Costabilisatoren und/oder
t) IR-Strahlung regulierende Verbindungen.

Zur Gruppe a) der 4,4-Diarylbutadiene zählen beispielsweise Verbindungen der Formel (aa)

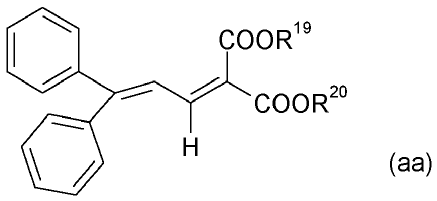

Die Verbindungen sind aus der EP-A-916 335 bekannt. Die Substituenten R¹⁹ und R²⁰ bedeuten, unabhängig voneinander, gleich oder verschieden, bevorzugt C₁-C₈-Alkyl und C₅-C₈-Cycloalkyl.

Zur Gruppe b) der Zimtsäureester zählen beispielsweise 4-Methoxyzimtsäure-2-isoamylester, 4-Methoxyzimtsäure-2-ethylhexylester, Methyl-α-methoxycarbonylcinnamat, Methyl-α-cyano-β-methyl-p-methoxycinnamat, Butyl-α-cyano-β-methyl-p-methoxy-cinnamat und Methyl-α-methoxycarbonyl-p-methoxycinnamat.

Zur Gruppe c) der Benzotriazole zählen beispielsweise 2-(2'-Hydroxyphenyl)-benzotriazole wie 2-(2'-Hydroxy-5'-methylphenyl)-benzotriazol, 2-(3',5'-Di-tert-butyl-2'-hydroxyphenyl)benzotriazol, 2-(5'-tert-Butyl-2'-hydroxyphenyl)-benzotriazol, 2-(2'-Hydroxy-5'-(1,1,3,3-tetramethylbutyl)phenyl)benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octyloxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol und 2-(3'-tert-Butyl-2'-hydroxy-5'-(2-isooctyloxy-carbonylethyl)-phenylbenzotriazol, 2,2'-Methylen-bis[4-(1,1,3,3-tetramethylbutyl)-6-benzotriazol-2-yl-phenol]; das Produkt der Veresterung von 2-[3'-tert-Butyl-5'-(2-methoxycarbonylethyl)-2'-hydroxyphenyl]-2H-benzotriazol mit Polyethylenglycol 300; [R-CH2CH2-COO(CH2)3]2, mit R = 3'-tert-Butyl-4'-hydroxy-5'-2H-benzotriazol-2-yl-phenyl und Gemische davon.

Zur Gruppe d) der Hydroxybenzophenone zählen beispielsweise 2-Hydroxybenzophenone wie 2-Hydroxy-4-methoxybenzophenon, 2,2'-Dihydroxy-4-methoxybenzophenon, 2,4-Dihydroxybenzophenon, 2,2',4,4'-Tetrahydroxybenzophenon, 2,2'-Dihydroxy-4,4'-dimethoxybenzophenon, 2,2'-Dihydroxy-4,4'-dimethoxybenzophenon, 2-Hydroxy-4-(2-ethylhexyloxy)benzophenon, 2-Hydroxy-4-(n-octyloxy)benzophenon, 2-Hydroxy-4-methoxy-4'-methylbenzophenon, 2-Hydroxy-3-carboxybenzophenon, 2-Hydroxy-4-methoxybenzophenon-5-sulfonsäure und deren Natriumsalz, 2,2'-Dihydroxy-4,4'-dimethoxybenzophenon-5,5'-bissulfonsäure und deren Natriumsalze.

Zur Gruppe e) der Diphenylcyanacrylate zählen beispielsweise Ethyl-2-cyan-3,3-diphenylacrylat, das beispielsweise im Handel unter dem Namen Uvinul® 3035 der Fa. BASF SE, Ludwigshafen erhältlich ist, 2-Ethylhexyl-2-cyan-3,3-diphenylacrylat, das beispielsweise im Handel als Uvinul® 3039 der Fa. BASF SE, Ludwigshafen, erhältlich ist und 1,3-Bis-[(2'-cyano-3',3'-diphenylacryloyl)oxy]-2,2-bis{[2'-cyano-3',3'-diphenyl-acryloyl)oxy]methyl}propan, das beispielsweise im Handel unter dem Namen Uvinul® 3030 der Fa. BASF SE, Ludwigshafen erhältlich ist.

Zur Gruppe f) der Oxamide zählen beispielsweise 4,4'-Dioctyloxyoxanilid, 2,2'-Diethoxyoxanilid, 2,2'-Dioctyloxy-5,5'-di-tert-butoxanilid, 2,2'-Didodecyloxy-5,5'-di-tert-butoxanilid, 2-Ethoxy-2'-ethyloxanilid, N,N'-Bis(3-dimethylaminopropyl)oxamid, 2-Ethoxy-5-tert-butyl-2'-ethoxanilid und dessen Mischung mit 2-Ethoxy-2'-ethyl-5,4'-di-tert-butoxanilid sowie Mischungen von ortho-, para-Methoxy-disubstituierten Oxaniliden und Mischungen von ortho- und para-Ethoxy disubstituierten Oxaniliden.

Zur Gruppe g) der 2-Phenyl-1,3,5-triazine zählen beispielsweise 2-(2-Hydroxyphenyl)-1,3,5-triazine wie 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propoxy)-phenyl]-4,6-bis(2,4-dimethyl)-1,3,5-triazin, 2-[2-Hydroxy-4-(2-hydroxy-3-octyloxy-propoxy)phenyl]-4,6-bis(2,4-dimethyl)-1,3,5-triazin, 2-[4-(Dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis-(2,4-dimethylphenyl)-1,3,5-triazin, 2-(2-Hydroxy-4-hexyloxyphenyl)-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin und 2-(2-Hydroxyphenyl)-4-(4-methoxyphenyl)-6-phenyl-1,3,5-triazin.

Die Gruppe h) der Antioxidantien umfasst beispielsweise:
Alkylierte Monophenole wie beispielsweise 2,6-Di-tert-butyl-4-methylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-tert-butyl-4-methoxymethylphenol, unverzweigte oder in der Seitenkette verzweigte Nonylphenole wie beispielsweise 2,6-Di-nonyl-4-methylphenol, 2,4-Dimethyl-6-(1-methylundec-1-yl)-phenol, 2,4-Dimethyl-6-(1-methylheptadec-1-yl)-phenol, 2,4-Dimethyl-6-(1-methyltridec-1-yl-)phenol und Gemische davon.

Alkylthiomethylphenole wie zum Beispiel 2,4-Dioctylthiomethyl-6-tert-butylphenol, 2,4-Dioctylthiomethyl-6-methylphenol, 2,4-Dioctylthiomethyl-6-ethylphenol, 2,6-Didodecylthiomethyl-4-nonylphenol.

Hydrochinone und alkylierte Hydrochinone wie zum Beispiel 2,6-Di-tert-butyl-4-methoxyphenol, 2,5-Di-tert-butylhydrochinon, 2,5-Di-tert-amylhydrochinon, 2,6-Diphenyl-4-octadecyloxyphenol, 2,6-Di-tert-butylhydrochinon, 2,5-Di-tert-butyl-4-hydroxyanisol, 3,5-Di-tert-butyl-4-hydroxyanisol, 3,5-Di-tert-butyl-4-hydroxyphenylstearat, Bis-(3,5-di-tert-butyl-4-hydroxyphenyl)adipat.

Tocopherole, wie zum Beispiel α-Tocopherol, β-Tocopherol, γ-Tocopherol, δ-Tocopherol und Gemische davon (Vitamin E).

Hydroxylierte Thiodiphenylether wie zum Beispiel 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis(6-tert-butyl-2-methylphenol), 4,4'-Thio-bis-(3,6-di-sec-amylphenol), 4,4'-Bis(2,6-dimethyl-4-hydroxyphenyl)disulfid.

Alkyliden-Bisphenole wie zum Beispiel 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)butan, 1,1-Bis(5-tert-butyl-4-hydroxy-2-methyl-phenyl)-3-n-dodecylmercaptobutan, Ethylenglycol-bis-[3,3-bis(3-tert-butyl-4-hydroxyphenyl)butyrat], Bis(3-tert-butyl-4-hydroxy-5-methyl-phenyl)dicyclopentadien, Bis[2-(3'-tert-butyl-2-hydroxy-5- methylbenzyl)-6-tert-butyl-4-methylphenyl]terephthalat, 1,1-Bis-(3, 5-dimethyl-2-hydroxyphenyl)butan, 2,2-Bis-(3,5-di-tert-butyl-4-hydroxyphenyl)propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

Benzylverbindungen wie zum Beispiel 3,5,3',5'-Tetra-tert-butyl-4,4'-dihydroxydibenzylether, Octadecyl-4-hydroxy-3,5-dimethylbenzylmercaptoacetat, Tridecyl-4-hydroxy-3,5-di-tert-butylbenzylmercaptoacetat, Tris(3,5-di-tert-butyl-4-hydroxybenzyl)amin, 1,3,5-Tri-(3,5-di-tert-butyl-4-hydroxybenzyl)-2,4,6-trimethylbenzol, Di-(3,5-Di-tert-butyl-4-hydroxybenzyl)sulfid, 3,5-Di-tert-butyl-4-hydroxybenzyl-mercapto-essigsäureisooctylester, Bis-(4-tert-butyl-3-hydroxy-2,6-dimethylbenzyl)dithiolterephthalat, 1,3,5-Tris-(3,5-di-tert-butyl-4-hydroxybenzyl)isocyanurat, 1,3,5-Tris-(4-tert-butyl-3-hydroxy-2, 6-dimethylbenzyl)isocyanurat, 3,5-Di-tert-butyl-4-hydroxybenzyl-phosphorsäuredioctadecylester und 3,5-Di-tert-butyl-4-hydroxybenzyl-phosphorsäuremonoethylester, Calciumsalz.

Hydroxybenzylierte Malonate wie zum Beispiel Dioctadecyl-2,2-bis-(3,5-di-tert butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)malonat, Bis[4-(1,1,3,3-tetramethylbutyl)phenyl]-2,2-bis(3,5-di-tert-butyl-4-hydroxybenzyl)malonat.

Hydroxybenzyl-Aromaten wie zum Beispiel 1,3,5-Tris-(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)phenol.

Triazinverbindungen wie zum Beispiel 2,4-Bis(octylmercapto)-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.

Benzylphosphonate wie zum Beispiel Dimethyl-2,5-di-tert-butyl-4-hydroxybenzylphosphonat, Diethyl-3,5-di-tert-butyl-4-hydroxybenzylphosphonat ((3,5-Bis(1,1-dimethylethyl)-4-hydroxyphenyl)methyl)Iphosphonsäurediethylester), Dioctade-cyl-3,5-di-tert-butyl-4-hydroxybenzylphosphonat, Dioctadecyl-5-tert-butyl-4-hydroxy-3-methylbenzylphosphonat, Calciumsalz des 3,5-Di-tert-butyl-4-hydroxybenzylphosphonsäure-monoethylesters.

Acylaminophenole wie zum Beispiel 4-Hydroxy-laurinsäureanilid, 4-Hydroxystearinsäureanilid, 2,4-Bis-octylmercapto-6-(3,5-tert-butyl-4-hydroxyanilino)-s-triazin und Octyl-N-(3,5-di-tert-butyl-4-hydroxyphenyl)-carbamat.

Ester der β-(3,5-Di-tert-butyl-4-hydroxyphenyl)propionsäure mit ein- oder mehrwertigen Alkoholen wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(Hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.

Ester der β-(5-tert-Butyl-4-hydroxy-3-methylphenyl)propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2] octan.

Ester der β-(3,5-Dicyclohexyl-4-hydroxyphenyl)propionsäure mit ein- oder mehrwertigen Alkoholen wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo [2.2.2]octan.

Ester der 3,5-Di-tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexanediol, Trimethylolpropan, 4-Hydroxymethyl-1-phospha-2,6,7-trioxabicyclo[2.2.2]octan.

Amide der β-(3, 5-Di-tert-butyl-4-hydroxyphenyl)propionsäure, wie z. B. N,N'-Bis(3,5-di-tert-butyl-4-hydroxyphenylpropionyl)-hexamethylendiamid, N,N'-Bis(3,5-di-tert-butyl-4-hydroxyphenylpropionyl)-trimethylendiamid, N,N'-Bis(3,5-di-tert-butyl-4-hydroxyphenylpropionyl)-hydrazid, N,N'-Bis[2-(3-[3,5-di-tert-butyl-4-hydroxyphenyl]-propionyloxy)ethyl]-oxamid (z. B. Naugard®XL-1 der Firma Uniroyal).

### Ascorbinsäure (Vitamin C)

Aminische Antioxidantien, wie zum Beispiel N,N'-Di-isopropyl-p-phenylendiamin, N,N'-Di-sec-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N, N'-Dicyclohexyl-p-phenylendiamin, N, N'-Diphenyl-p-phenylendiamin, N,N'-Bis(2-naphthyl)-p-phenylendiamin, N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-(4-tert-Octylphenyl)-1-naphthylamin, N-Phenyl-2-naphthylamin, octyliertes Diphenylamin, zum Beispiel p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylaminophenol, Bis-(4-methoxyphenyl)amin, 2,6-Di-tert-butyl-4-dimethylaminomethylphenol, 2,4'-Diaminodiphenylmethan, 4,4'-Diaminodiphenylmethan, N,N,N',N'-Tetramethyl-4,4'-diaminodiphenylmethan, 1,2-Bis-[(2-methylphenyl)amino]ethan,1,2-Bis(phenylamino)-propan, (o-Tolyl)-biguanid, Bis[4-(1',3'-dimethylbutyl)phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyldiphenylaminen, Gemisch aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, 2,2,6,6-Tetramethylpiperidin-4-on, 2,2,6,6 Tetramethylpiperidin-4-ol, das Dimethylsuccinat-Polymer mit 4-Hydroxy-2,2,6,6-tetramethyl-1-piperidinethanol [CAS Nummer 65447-77-0], (beispielsweise Tinuvin® 622 der Fa. Ciba Specialty Chemicals, Inc.), Polymer of 2,2,4,4-Tetramethyl-7-oxa-3,20-diaza-dispiro[5.1.11.2]-heeicosan-21-on und Epichlorhydrin [CAS-No.: 202483-55-4], beispielsweise (Hostavin® N 30 der Fa. Clariant).

Zu Gruppe i) der Nickelverbindungen gehören zum Beispiel Nickel-Komplexe des 2,2'-Thio-bis-[4-(1,1,3,3-tetramethylbutyl)phenols], wie der 1:1 oder 1:2 Komplex, gegebenenfalls mit zusätzlichen Liganden wie n-Butylamin, Triethanolamin oder N-Cyclohexyldiethanolamin, Nickeldibutyldithiocarbamat, Nickelsalze von 4-Hydroxy-3,5-di-tert-butylbenzylphosphonsäuremonoalkylester wie z. B. der Methyl- oder Ethylester, Nickelkomplexe von Ketoximen wie z. B. von 2-Hydroxy-4-methylphenylundecylketoxim, Nickelkomplex von 1-Phenyl-4-lauroyl-5-hydroxypyrazol, gegebenenfalls mit zusätzlichen Liganden.

Zur Gruppe j) der sterisch gehinderten Amine, die nicht eine oder mehrere Gruppen der allgemeinen Formel (I) enthalten, gehören zum Beispiel 4-Hydroxy-2,2,6,6-tetramethylpiperidin, 1-Allyl-4-hydroxy-2,2,6,6-tetramethylpiperidin, 1-Benzyl-4-hydroxy-2,2,6,6-tetramethylpiperidin, Bis(2,2,6,6-tetramethyl-4-piperidyl)sebacat, Bis(2,2,6,6-tetramethyl-4-piperidyl)succinat, Bis(1,2,2,6,6-pentamethyl-4-piperidyl)sebacat, Bis(1-octyloxy-2, 2,6,6-tetramethyl-4-piperidyl)sebacat, Bis(1,2,2,6,6-pentamethyl-4-piperidyl)-n-butyl-3,5-di-tert-butyl-4-hydroxybenzylmalonat (n-Butyl-3,5-di-tert-butyl-4-hydroxy-benzyl-malonsäure-bis(1,2,2,6,6-pentamethylpiperidyl)-ester), Kondensationsprodukt aus 1-(2-Hydroxyethyl)-2,2,6,6-tetramethyl-4-hydroxypiperidin und Bernsteinsäure, lineare oder cyclische Kondensationsprodukte aus N,N'-Bis(2,2,6,6-tetramethyl-4-piperidyl)hexamethylendiamin und 4-tert-Octylamino-2,6-dichlor-1,3,5-triazin, Tris(2,2,6,6-tetramethyl-4-piperidyl)-nitrilotriacetat, Tetrakis(2,2,6,6-tetramethyl-4-piperidyl)-1,2,3,4-butan-tetracarboxylat, 1,1'-(1, 2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert butylbenzyl)malonat, 3-n-Octyl-7,7,9,9-tetramethyl-1,3,8-triazaspiro [4.5]decan-2,4-dion, Bis-(1-octyloxy-2,2,6,6-tetramethylpiperidyl)-sebacat, Bis-(1-octyloxy-2,2,6,6-tetramethyl-piperidyl)-succinat, lineare oder cyclische Kondensationsprodukte von N,N'-Bis-(2,2,6,6-tetramethyl-4-piperidyl)hexamethylendiamin und 4-Morpholino-2,6-dichlor-1,3,5-triazin, Kondensationsprodukt von 2-Chlor-4,6-bis(4-n-butylamino-2, 2,6,6-tetramethylpiperidyl)-1,3,5-triazin und 1,2-Bis(3-aminopropylamino)ethan, Kondensationsprodukt von 2-Chlor-4,6-di-(4-n-butylamino-1,2,2,6,6-pentamethylpiperidyl)-1,3,5-triazin und 1,2-Bis-(3-aminopropylamino)ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)pyrrolidin-2,5-dion, Gemisch von 4-Hexadecyloxy-und 4-Stearyloxy-2,2,6,6-tetramethylpiperidin, Kondensationsprodukt aus N,N'-Bis(2,2,6,6-tetramethyl-4-piperidyl)hexamethylendiamin und 4-Cyclohexylamino-2,6-dichlor-1,3,5-triazin, Kondensationsprodukt aus 1,2-Bis(3-aminopropylamino)ethan und 2,4,6-Trichlor-1,3,5-triazin sowie 4-Butylamino-2,2,6,6-tetramethylpiperidin (CAS Reg. No. [136504-96-6]); N-(2,2,6,6-Tetramethyl-4-piperidyl)-n-dodecylsuccinimid, N-(1,2,2,6,6-Pentamethyl-4-piperidyl)-n-dodecylsuccinimid, 2-Undecyl-7,7,9,9-tetramethyl-1-oxa-3,8-diaza-4-oxospiro[4, 5]decan, Reaktionsprodukt von 7,7,9,9-Tetramethyl-2-cycloundecyl-1-oxa-3,8-diaza-4-oxospiro[4, 5]decan und Epichlorhydrin, 1,1-Bis(1,2,2,6,6-pentamethyl-4-piperidyloxycarbonyl)-2-(4-methoxyphenyl)ethen, Diester der 4-Methoxy-methylenmalonsäure mit 1,2,2,6,6-Pentamethyl-4-hydroxypiperidin, Poly[methylpropyl-3-oxo-4-(2,2,6,6-tetramethyl-4-piperidyl)]siloxan, 1-(2-Hydroxy-2-methylpropoxy)-4-octadecanoyloxy-2,2,6,6-tetramethylpiperidin, 1-(2-Hydroxy-2-methylpropoxy)-4-hexadecanoyloxy-2, 2,6,6-tetramethylpiperidin, das Reaktionsprodukt aus 1-Oxyl-4-hydroxy-2,2,6,6-tetramethylpiperidin und einem Kohlenstoffrest von t-Amylalkohol, 1-(2-Hydroxy-2-methylpropoxy)-4-hydroxy-2,2,6,6-tetramethylpiperidin, 1-(2-Hydroxy-2-methylpropoxy)-4oxo-2,2,6,6-tetramethylpiperidin, Bis(1-(2-hydroxy-2-methylpropoxy)-2,2,6,6-tetramethylpiperidin-4-yl)sebacat, Bis(1-(2-hydroxy-2-methylpropoxy)-2, 2,6,6-tetramethylpiperidin-4-yl)adipat, Bis(1-(2-hydroxy-2-methylpropoxy)-2, 2,6,6-tetramethylpiperidin-4-yl)succinat, Bis(1-(2-hydroxy-2-methylpropoxy)-2, 2,6,6-tetramethylpiperidin-4-yl)glutarat, 2,4-bis{N[1-(2-hydroxy-2-methylpropoxy)-2,2,6,6-tetramethylpiperidin-4-yl]-N-butylamino}-6-(2hydroxyethylamino)-s-triazin, Hexahydro-2,6-bis(2,2,6,6-tetramethyl-4-piperidyl)-1H,4H,5H,8H-2,3a,4a,6,7a,8a-hexaazacyclopenta[def]fluoren-4,8-dion (z. B. Uvinul® 4049 der Fa. BASF SE, Ludwigshafen), Poly[[6-[(1,1,3,3-tetramethylbutyl)amino]-1,3,5-triazin-2,4-diyl][(2,2,6,6-tetramethyl-4-piperidinyl)imino]1,6-hexandiyl[(2,2,6,6-tetramethyl-4-piperidinyl)imino]]) [CAS Nr. 71878-19-8], 1,3,5-Triazin-2,4,6-triamin,N,N"'-[1,2-ethan-diyl-bis [[4,6-bis-[butyl(1,2,2,6,6-pentamethyl-4-piperidinyl)amino]-1,3,5-triazin-2-yl]imino]-3,1-propandiyl]]bis[N',N"-dibutyl-N',N"-bis(1,2,2,6,6-pentamethyl-4-piperidinyl)- (CAS Nr. 106990-43-6) (z. B. Chimassorb 119 der Fa. Ciba Specialty Chemicals, Inc.).

Zur Gruppe k) der Metalldesaktivatoren gehören zum Beispiel N,N'-Diphenyloxalsäurediamid, N-Salicylal-N'-salicyloyl-hydrazin, N,N'-Bis(salicyloyl)hydrazin, N, N'-Bis(3,5-di-tert-butyl-4-hydroxyphenylpropionyl)hydrazin, 3-Salicyloylamino-1,2,4-triazol, Bis(benzyliden)-oxalyldihydrazid, Oxanilid, Isophthaloyldihydrazid, Sebacoylbisphenylhydrazid, N, N'-Diacetyladipinsäuredihydrazid, N,N'-Bis(salicyloyl)oxalsäuredihydrazid, N,N'-Bis(salicyloyl)thiopropionyldihydrazid.

Zur Gruppe I) der Phosphite und Phosphonite gehören zum Beispiel Triphenylphosphit, Diphenylalkylphosphite, Phenyldialkylphosphite, Tris(nonylphenyl)phosphit, Trilaurylphosphit, Trioctadecylphosphit, Distearylpentaerythritdiphosphit, Tris(2,4-di-tert-butylphenyl)phosphit, Diisodecylpentaerythritdiphosphit, Bis(2,4-di-tert-butylphenyl)pentaerythritdiphosphit, Bis(2,6-di-tert-butyl-4-methylphenyl)-pentaerythritdiphosphit, Diisodecyloxypentaerythritdiphosphit, Bis(2,4-di-tert-butyl-6-methylphenyl)-pentaerythritdiphosphit, Bis(2,4,6-tris(tert-butylphenyl)pentaerythrit-diphosphit, Tristearylsorbittriphosphit, Tetrakis-(2,4-di-tert-butylphenyl)-4,4'-biphenylendiphosphonit, 6-Isooctyloxy-2,4,8,10-tetra-tert-butyl-dibenz[d,f][1,3,2]di-oxaphosphepin, 6-Fluor-2,4,8,10-tetra-tert-butyl-12-methyl-dibenz[d,g][1,3,2]di-oxaphosphocin, Bis(2,4-di-tert-butyl-6-methylphenyl)methylphosphit, Bis(2,4-di-tert-butyl-6-methylphenyl)ethylphosphit, 2,2',2"-Nitrilo[triethyl-tris(3,3', 5,5'-tetra-tert-butyl-1,1'-biphenyl-2,2'-diyl)phosphit], 2- Ethylhexyl-(3,3', 5,5'-tetra-tert-butyl-1,1'-biphenyl-2,2'-diyl)phosphit.

Zur Gruppe m) der Hydroxylamine gehören zum Beispiel N, N-Dibenzylhydroxylamin, N, N-Diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecyl-hydroxylamin, N-Hexadecyl-N-octadecylhydroxylamin, N-Heptadecyl-N-octadecylhydroxylamin, N-Methyl-N-octadecylhydroxylamin und N, N-Dialkylhydroxylamin aus hydrierten Talgfettaminen.

Zur Gruppe n) der Nitrone gehören zum Beispiel N-Benzyl-α-phenylnitron, N-Ethyl-α-methylnitron, N-Octyl-α-heptylnitron, N-Lauryl-α-undecylnitron, N-Tetradecyl-α-tridecylnitron, N-Hexadecyl-α-pentadecylnitron, N-Octadecyl-α-heptadecylnitron, N-Hexadecyl-α-heptadecylnitron, N-Ocatadecyl-α-pentadecylnitron, N-Heptadecyl-α-heptadecylnitron, N-Octadecyl-α-hexadecylnitron, N-Methyl-α-heptadecylnitron und Nitrone, abgeleitet von N,N-Dialkylhydroxylaminen hergestellt aus hydrierten Talkfettaminen.

Zur Gruppe o) der Aminoxide gehören zum Beispiel Aminoxidderivate wie sie in den U.S. Patenten Nr. 5,844,029 und 5,880,191 beschrieben sind, Didecylmethylaminoxid, Tridecylaminoxid, Tridodecylaminoxid und Trihexadecylaminoxid.

Zur Gruppe p) der Benzofuranone und Indolinone gehören zum Beispiel die in den US-Patenten 4,325,863; 4,338,244; 5,175,312; 5,216,052; 5,252,643; in der DE-A-4316611; in der DE-A-4316622; in der DE-A-4316876; in der EP-A-0589839 oder EP-A-0591102 beschriebenen oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]benzofuran-2-on, 3,3'-Bis[5,7-di-tert-butyl-3-(4-[2-hydroxyethoxy]phenyl)benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxyphenyl)-5,7-di-tert-butyl-benzofuran-2-on, 3-(3,4-Dimethylphenyl)-5,7-di-tert-butyl-benzofuran-2-on, Irganox HP-136 der Firma Ciba Specialty Chemicals, und 3-(2,3-Dimethylphenyl)-5,7-di-tert-butyl-benzofuran-2-on.

Zur Gruppe q) der Thiosynergisten gehören zum Beispiel Dilaurylthiodipropionat oder Distearylthiodipropionat.

Zur Gruppe r) der peroxidzerstörende Verbindungen gehören zum Beispiel Ester der β-Thiodipropionsäure, zum Beispiel der Lauryl-, Stearyl-, Myristyl- oder Tridecylester, Mercaptobenzimidazol oder das Zinksalz des 2-Mercaptobenzimidazols, Zinkdibutyldithiocarbamat, Dioctadecyldisulfid, Pentaerythrit-tetrakis(β-dodecylmercapto)-propionat.

Zur Gruppe s) der basischen Costabilisatoren gehören zum Beispiel Melamin, Polyvinylpyrrolidon, Dicyandiamid, Triallylcyanurat, Harnstoffderivate, Hydrazinderivative, Amine, Polyamide, Polyurethane, Alkali- und Erdalkalisalze höherer Fettsäuren, zum Beispiel Calciumstearat, Zinkstearat, Magnesiumbehenat, Magnesiumstearat, Natriumricinoleat und Kaliumpalmitat, Antimonbrenzcatechinat oder Zinkbrenzcatechinat.

Zur Gruppe t) der IR-Strahlung regulierenden Verbindungen gehören IR-Absorber, beispielsweise anorganische oder organische IR-Absorber, wie Antimon-Zinnoxid (ATO), Indium-Zinnoxid (ITO), Lumogen® IR 1050 der BASF SE. Weiterhin gehören zur Gruppe t) für IR-Strahlung durchlässige Additive wie Perlyenverbindungen, beispielsweise Lumogen® black FK 4280 und 4281 der Fa. BASF SE. Zur Gruppe t) gehören auch IR-Strahlung reflektierende Stoffe, insbesondere Pigmente enthaltend Eisen- und Chromoxide, beispielsweise Sicopal® Black K 0095, insbesondere für den Einsatz in Kunststoffen und Pigmente enthaltend Perylene, beispielsweise Paliogen® Black S 0084 und 0086, insbesondere für den Einsatz in Lacken.

Weiterhin kommen als geeignete Additive die üblichen Zusatzstoffe, wie z. B. Pigmente, Farbstoffe, Nukleierungsmittel, Füll- oder Verstärkungsmittel, Beschlagverhinderungsmittel, Biozide und Antistatika, in Betracht.

Geeignete Pigmente sind anorganische Pigmente, beispielsweise Titandioxid in seinen drei Modifikationen Rutil, Anatas oder Brookit, Ultramarinblau, Eisenoxide, Bismutvanadate oder Ruß sowie die Klasse der organischen Pigmente, beispielsweise Verbindungen aus der Klasse der Phthalocyanine, Perylene, Azoverbindungen, Isoindoline, Chinophthalone, Diketopyrolopyrole, Chinacridone, Dioxazine, Indanthrone.

Unter Farbstoffen sind alle Farbmittel zu verstehen, die sich im verwendeten Kunststoff vollständig lösen bzw. in einer molekulardispersen Verteilung vorliegen und somit zur hochtransparenten, nichtstreuenden Einfärbung von Polymeren verwendet werden können. Ebenfalls als Farbstoffe sind organische Verbindungen anzusehen, die eine Fluoreszenz im sichtbaren Teil des elektromagnetischen Spektrums aufweisen wie Fluoreszenzfarbstoffe.

Geeignete Nukleierungsmittel umfassen zum Beispiel anorganische Stoffe, beispielsweise Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z. B. 4-tert-Butylbenzoesäure, Adipinsäure, Diphenylessigsäure, Natriumsuccinat oder Natriumbenzoat; polymere Verbindungen, wie beispielsweise ionische Copolymerisate ("lonomere").

Geeignete Füll- oder Verstärkungsstoffe umfassen zum Beispiel Calciumcarbonat, Silikate, Talk, Mica, Kaolin, Glimmer, Bariumsulfat, Metalloxide und -hydroxide, Ruß, Graphit, Holzmehl und Mehle oder Fasern anderer Naturprodukte, synthetische Fasern. Als Beispiele für faserförmige bzw. pulverförmige Füllstoffe kommen außerdem Kohlenstoff- oder Glasfasern in Form von Glasgeweben, Glasmatten oder Glasseidenrovings, Schnittglas, Glaskugeln sowie Wollstonit in Betracht. Die Einarbeitung von Glasfasern kann sowohl in Form von Kurzglasfasern als auch in Form von Endlosfasern (Rovings) erfolgen.

Geeignete Antistatika sind beispielsweise Aminderivate wie N,N-Bis(hydroxyalkyl)alkylamine oder -alkylenamine, Polyethylenglycolester und -ether, ethoxylierte Carbonsäureester- und -amide und Glycerinmono- und - distearate, sowie deren Mischungen.

Das Gewichtsverhältnis der sterisch gehinderten Amine, enthaltend eine oder mehrere Gruppen der allgemeinen Formel (I), insbesondere sterisch gehinderten Amine der Formel (I-a) bis (I-t), zu der Gesamtmenge an Additiven beträgt, falls Additive vorhanden sind, im Allgemeinen von 100:1 bis 2:1, bevorzugt von 50:1 bis 5:1, besonders bevorzugt von 30:1 bis 7:1 insbesondere bevorzugt ist eine Mischung im Gewichtsverhältnis von etwa 10:1.

Eine besonders vorteilhafte Ausführungsform der erfindungsgemäßen Verwendung umfasst neben dem Einsatz von sterisch gehinderten Amine, enthaltend eine oder mehrere Gruppen der allgemeinen Formel (I), insbesondere sterisch gehinderten Amine der Formel (I-a) bis (I-t), die Verwendung von Flammschutzmitteln. Die Flammschutzmittel werden in der Regel ausgewählt aus: flammhemmenden Mitteln auf Melaminbasis und/oder Ammoniumpolyphosphat, Bis-(hexachlorcyclopentadieno)-cyclooctan,Tris-(2,3-dibrompropy1)-isocyanurat, Ethylen-bis-tetrabromphthalimid, 1,2,5,6,9,10-Hexabromcyclododecan und/oder Ethan-1,2-bis-(pentabromphenyl). Selbstverständlich ist auch der Einsatz von Mischungen verschiedener Flammschutzmittel möglich.

Ebenfalls können halogenierte flammhemmende Mittel ausgewählt werden aus folgenden Verbindungen: organische, aromatische, halogenierte Verbindungen, wie halogenierte Benzole, Biphenyle, Phenole, Ether oder Ester davon, Bisphenole, Diphenyloxide, aromatische Carbonsäuren oder Polysäuren, Anhydride, Amide oder Imide davon; organische, cycloaliphatische oder polycycloaliphatische, halogenierte Verbindungen; und organische aliphatische, halogenierte Verbindungen, wie halogenierte Paraffine, Oligo- oder Polymere, Alkylphosphate oder Alkylisocyanurate. Diese Komponenten sind aus dem Stand der Technik bekannt, vgl. US 4,579,906 (z. B. Spalte 3, Zeilen 30-41), US 5,393,812; vgl. auch Plastics Additives Handbook, Hrsg. H. Zweifel, 5. Auflag., Hanser Verlag, München 2001, S. 681-698. Bei den halogenierten flammhemmenden Mitteln kann es sich beispielsweise um eine chlorierte oder bromierte Verbindung handeln, die z. B. unter folgenden Verbindungen ausgewählt ist: Chloralkylphosphatester (ANTIBLAZE® AB-1 00, Albright & Wilson; FYROL® FR-2, Akzo Nobel), polybromiertes Diphenyloxid (DE-6OF, Great Lakes Corp.), Decabromdiphenyloxid (DBDPO; SAYTEX® 102E), Tris-[3-brom-2,2-bis-(brommethyl)-propyl-phosphat (PB 370, FMC Corp.), Bis-(2,3-dibrompropylether) von Bisphenol A (PE68), bromiertes Epoxyharz, Ethylen-bis-(tetrabromphthalimid) (SAYTEX® BT-93), Bis-(hexachlorcyclopentadieno)-cyclooctan (DECLORANE PLUS®), chlorierte Paraffine, 1,2-Bis-(tribromphenoxy)-ethan (FF680), Tetrabrombisphenol A (SAYTEX® RB 100), Ethylen-bis-(dibrom-norbornandicarboximid) (SAYTEX® BN-451), Bis-(hexachlorcyclopentadieno)-cyclooctan, Tris-(2,3-dibrompropy1)-isocyanurat, Ethylenbistetrabromphthalimid. Bromierte flammhemmende Mittel werden bevorzugt. Besonders bevorzugte flammhemmende Mittel sind: flammhemmende Mittel auf der Basis von Melamin und/oder Ammoniumpolyphosphat, Bis-(hexachlorcyclopentadieno)-cyclooctan, Tris-(2,3-dibrompropy1)-isocyanurat, Ethylenbistetrabromphthalimid, 1,2,5,6,9,10-Hexabromcyclododecan, Ethan-1,2-bis-(pentabromphenyl), Tris-(3-brom-2,2-(brommethy1)-propy1)-phosphat.

Weitere bevorzugte flammhemmende Mittel sind chloriertes Paraffin, 1,2-bis(tribromphenoxy)ethan, Decabrombiphenyl, Decabromdiphenylethan, Decabromdiphenylether, Octabromdiphenylether, Ethylen-bis-(5,6-dibromnorbornan-2,3-dicarboximid), Ethylen-bis(tetrabrompthalimid), Hexabromcyclododecan, Tetrabrombisphenol A -bis-(2,3-dibrompropyl)- ether. Besonders bevorzugt sind Decabromdiphenylethan und Hexabromcyclododecan.

Das flammhemmende Mittel ist häufig in einer Menge von 0,5 bis 50 Gew.-%, bezogen auf das unbelebte organische Materialien, beispielsweise Kunststoffe (Polymere) oder Lacke, insbesondere Kunststoffe enthalten. Das Verhältnis des flammhemmenden Mittels zu den sterisch gehinderten Aminen der allgemeinen Formel (I), insbesondere sterisch gehinderten Amine der Formel (I-a) bis (I-t), liegt vorzugsweise im Bereich von 20 : 1 bis 250 : 1.

Eine besonders vorteilhafte Ausführungsform der erfindungsgemäßen Verwendung umfasst wie oben bereits erwähnt neben dem Einsatz von sterisch gehinderten Amine, enthaltend eine oder mehrere Gruppen der allgemeinen Formel (I), insbesondere sterisch gehinderten Amine der Formel (I-a) bis (I-t), die Verwendung von halogenierten Flammschutzmitteln. Der Vorteil dieser Kombination ist, dass die sterisch gehinderten Amine, enthaltend eine oder mehrere Gruppen der allgemeinen Formel (I), insbesondere sterisch gehinderten Amine der Formel (I-a) bis (I-t), in Gegenwart der halogenierten Flammschutzmittel ihre Wirkung nicht verlieren, während sterisch gehinderte Amine, die auf den entsprechenden N-H oder N-Alkyl-Derivaten basieren durch die halogenierten Flammschutzmittel desaktiviert werden.

Ein weiterer Gegenstand der Erfindung ist ein Verfahren zur Stabilisierung von unbelebten organischen Materialien, insbesondere ein Verfahren zum Schutz von unbelebten organischen Materialien gegen die negativen Auswirkungen von elektromagnetischer Strahlung, insbesondere Licht, Wärme oder Radikalen, insbesondere Sauerstoff, wobei den Materialien sterisch gehinderte Amine, enthaltend eine oder mehrere Gruppen der allgemeinen Formel (I), insbesondere sterisch gehinderte Amine der allgemeinen Formeln (I-a) bis (I-q), in einer für die Stabilisierung effektiven Menge zugesetzt werden, bevorzugt in einer Menge von 0,01 bis 5 Gew.-%, vorzugsweise von 0,02 bis 1 Gew.-%, bezogen auf das unbelebte organische Material.

Ein weiterer Gegenstand der Erfindung sind ausgewählte sterisch gehinderte Amine der allgemeinen Formel (I-b) wobei R⁴, R⁵, R⁶, R⁷ alle Methyl sind, R⁸ Wasserstoff, R¹⁰ Formyl, p = 2, R¹¹ Hexamethylen, R¹ Wasserstoff, R² Wasserstoff und R³ Phenyl ist, wobei der Phenylrest R³ jeweils an beliebiger Position, bis zu fünfmal, bevorzugt nicht mehr als viermal und besonders bevorzugt nicht mehr als dreimal, durch Hydroxy, Amino, Mono- oder Di-C₁-C₂₀-Alkylamino, Nitro, Cyano, CO₂M¹, CONM¹M², SO₃M¹, C₁-C₂₀-Alkyl, C₁-C₂₀-Alkoxy, Aryl, Heterocyclus, Heteroatome oder Halogen substituiert sein kann und M¹, M² unabhängig voneinander, gleich oder verschieden, H oder C₁-C₂₀-Alkyl sind. Besonders bevorzugt ist jedoch der Phenylrest R³ unsubstituiert.

Ein weiterer Gegenstand der Erfindung sind ausgewählte sterisch gehinderte Amine der allgemeinen Formel (I-q) wobei R²⁰ ein Gemisch aus C₁₈-C₂₂-Alkylgruppen, insbesondere ein Gemisch aus C₁₈-Alkyl, C₂₀-Alkyl und C₂₂-Alkyl ist. Besonders bevorzugt sind hierbei sterische Amine der Formel (I-q), für die gilt, dass R²⁰ eine Mischung aus C₁₈-C₂₂-Alkylgruppen, insbesondere ein Gemisch aus C₁₈-Alkyl, C₂₀-Alkyl und C₂₂-Alkyl ist und die Mehrzahl (mehr als 50%) der Substituenten R²³ einer Gruppe -OCR¹R²R³ entsprechen. Hierbei sind
- R¹, R²: unabhängig voneinander, gleich oder verschieden, H, C₁-C₂₀-Alkyl, C₃-C₁₅-Cycloalkyl, Aryl, Hetaryl, Heterocyclus,
- R³: Aryl, Hetaryl,
- R⁴, R⁵, R⁶, R⁷: unabhängig voneinander, gleich oder verschieden, C₁-C₂₀-Alkyl, oder R⁴ und R⁶ oder R⁵ und R⁷ zusammen eine Tetramethylen- oder Pentamethylengruppe,
- R²¹, R²²: unabhängig voneinander, gleich oder verschieden, H, C₁-C₂₀-Alkyl, Aryl, C₁-C₂₀-Alkoxy, C2-C20-Alkenyl.

Bevorzugt sind hierbei
- R¹, R²: unabhängig voneinander, gleich oder verschieden, H, C₁-C₂₀-Alkyl,
- R³: Aryl,
- R⁴, R⁵, R⁶, R⁷: unabhängig voneinander, gleich oder verschieden, C₁-C₂₀-Alky.

Besonders bevorzugt sind hierbei
- R¹, R²: H,
- R³: Aryl,
- R⁴, R⁵, R⁶, R⁷: Methyl.

Ein weiterer Gegenstand der Erfindung sind ausgewählte sterisch gehinderte Amine der allgemeinen Formel (I-r), wobei
- R⁴, R⁵, R⁶, R⁷: unabhängig voneinander, gleich oder verschieden, C₁-C₂₀-Alkyl, oder R⁴ und R⁶ oder R⁵ und R⁷ zusammen eine Tetramethylen- oder Pentamethylengruppe, bevorzugt C₁-C₄-Alkyl, insbesondere bevorzugt alle Methyl
- R23: -OC(R¹R²R³)
- R¹, R²: unabhängig voneinander, gleich oder verschieden, H, C₁-C₂₀-Alkyl, C₃-C₁₅-Cycloalkyl, Aryl, Hetaryl, Heterocyclus, bevorzugt beide H,
- R³: Aryl, Hetaryl, bevorzugt Aryl,
- R²⁰: C₁-C₃₀-Alkyl, Aryl, Gemisch verschiedener C₁-C₃₀-Alkyl,
- R²¹: unabhängig voneinander, gleich oder verschieden, H, C₁-C₂₀-Alkyl, Aryl, C₁-C₂₀-Alkoxy, C₂-C₂₀-Alkenyl, bevorzugt C₁₆-C₂₂-Alkyl,
- R²⁴, R²⁵: unabhängig voneinander, gleich oder verschieden, C₁-C₂₄-Alkyl, bevorzugt C₁₀-C₂₄-Alkyl, besonders bevorzugt C₁₄-C₂₂-Alkyl, ganz besonders bevorzugt C₁₈-C₂₂-Alkyl,
- X²: O, NH
sind.

Ein weiterer Gegenstand der Erfindung sind ausgewählte sterisch gehinderte Amine der allgemeinen Formel (I-s), wobei
- R⁴, R⁵, R⁶, R⁷: unabhängig voneinander, gleich oder verschieden, C₁-C₂₀-Alkyl, oder R⁴ und R⁶ oder R⁵ und R⁷ zusammen eine Tetramethylen- oder Pentamethylengruppe, bevorzugt C₁-C₄-Alkyl, insbesondere bevorzugt alle Methyl
- R23: -OC(R¹R²R³)
- R¹, R²: unabhängig voneinander, gleich oder verschieden, H, C₁-C₂₀-Alkyl, C₃-C₁₅-Cycloalkyl, Aryl, Hetaryl, Heterocyclus, bevorzugt beide H,
- R³: Aryl, Hetaryl, bevorzugt Aryl,
- R²⁴: unabhängig voneinander, gleich oder verschieden, C₁-C₂₄-Alkyl, bevorzugt C₁₀-C₂₄-Alkyl, besonders bevorzugt C₁₄-C₂₂-Alkyl, ganz besonders bevorzugt C₁₈-C₂₂-Alkyl,
sind.

Ein weiterer Gegenstand der Erfindung sind ausgewählte sterisch gehinderte Amine der allgemeinen Formel (I-t), wobei
- R⁴, R⁵, R⁶, R⁷: unabhängig voneinander, gleich oder verschieden, C₁-C₂₀-Alkyl, oder R⁴ und R⁶ oder R⁵ und R⁷ zusammen eine Tetramethylen- oder Pentamethylengruppe, bevorzugt C₁-C₄-Alkyl, insbesondere bevorzugt alle Methyl
- R²³: -OC(R¹R²R³)
- R¹, R²: unabhängig voneinander, gleich oder verschieden, H, C₁-C₂₀-Alkyl, C₃-C₁₅-Cycloalkyl, Aryl, Hetaryl, Heterocyclus, bevorzugt beide H,
- R³: Aryl, Hetaryl, bevorzugt Aryl,
- R²⁴, R²⁵: unabhängig voneinander, gleich oder verschieden, C₁-C₂₄-Alkyl, bevorzugt C₁₀-C₂₄-Alkyl, besonders bevorzugt C₁₃-C₂₂-Alkyl, ganz besonders bevorzugt C₁₈-C₂₂-Alkyl,
- X²: O, N H
sind. Insbesondere bevorzugt sind hierbei auch sterisch gehinderte Amine der allgemeinen Formel (I-t) mit R²⁴ und R²⁵ gleich oder verschieden C₁₄-C₁₆-Alkyl.

Die Herstellung dieser ausgewählten sterisch gehinderten Amine erfolgt nach den oben beschriebenen Verfahren zur Herstellung der sterisch gehinderten Amine, enthaltend eine oder mehrere Gruppen der allgemeinen Formel (I) oder sterisch gehinderter Amine der Formel (I-a) bis (I-t).

Ein weiterer Gegenstand der Erfindung sind Materialien, insbesondere unbelebte organische Materialien, enthaltend die oben beschriebenen ausgewählten sterisch gehinderten Amine. Bevorzugt sind diese ausgewählten sterisch gehinderten Amine in einer Menge von 0,01 bis 5 Gew.-%, vorzugsweise von 0,02 bis 1 Gew.-%, bezogen auf das unbelebte organische Material enthalten. Ebenfalls bevorzugt sind die stabilisierten unbelebten organischen Materialien Kunststoffe oder Lacke, insbesondere Kunststoffe wie oben beschrieben. Ganz bevorzugt sind diese Kunststoffe Agrarfolien, PVC oder flammgeschützte Thermoplasten.

Beispiele:
Raumtemperatur: 21 °C

### Beispiel 1:

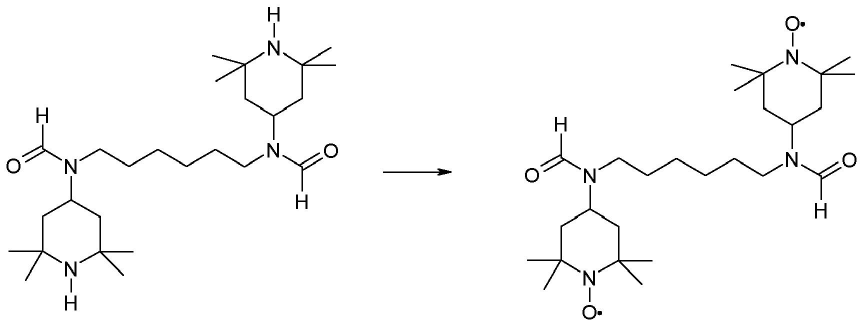

Zu einer Lösung von N,N'-Bisformyl-N,N'-bis-(2,2,6,6-tetramethyl-4-piperidinyl)-Hexamethylendiamin (Uvinul 4050 H der BASF SE) (49.00 g) in MeOH (480 mL) und H₂O (160 mL) wurden Na₂WO₄ (9.15 g) und H₂O₂ (30%, 65 mL) gegeben und 24 Stunden bei Raumtmeperatur gerührt. Nach dem Absaugen wurden 50 g (96%) des Diradikals als orangefarbener Feststoff erhalten. Das Produkt wurde ohne weitere Aufreinigung weiterverwendet.

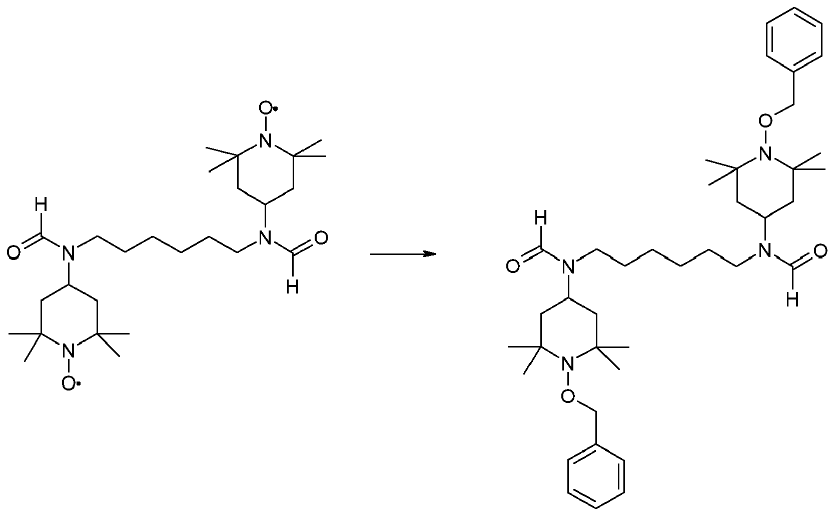

Eine Mischung aus Benzylbromid (7.12 g), Nitroxylradikal aus Beispiel 1 (12 g), Kupferpulver (2.78 g), Cu(OTf)₂ (151 mg) und 4,4'-Ditertbutyl-2,2'Bipyridyl (im folgenden als dTbPy bezeichnet) (0.45 g) in Benzol (70 m L) wurden entgast und für 18 Stunden zum Rückfluss erhitzt. Nach Abkühlen auf Raumtemperatur wurde vom Rückstand abfiltriert. Das Filtrat wurde eingeengt und mittels Flash-Chromatographie über Kieselgel gereinigt (Eluent 1:20 Ethylacetat/Petrolether). Man erhielt das Produkt als weißen Feststoff. ¹H NMR (300 MHz, CDCl₃) δ 1.24-1.34 (m, 30 H), 1.62 (s, 6 H), 1.81 (dd, *J* = 9.6, 22.8 Hz, 2 H), 3.23-3.40 (m, 4 H), 3.60-3.67 (m, 1.4 H), 4.51-4.56 (m, 0.7 H), 4.82 (s, 4 H), 7.27-7.36 (m, 10 H), 8.10-8.18 (m, 2 H).

### Beispiel 2:

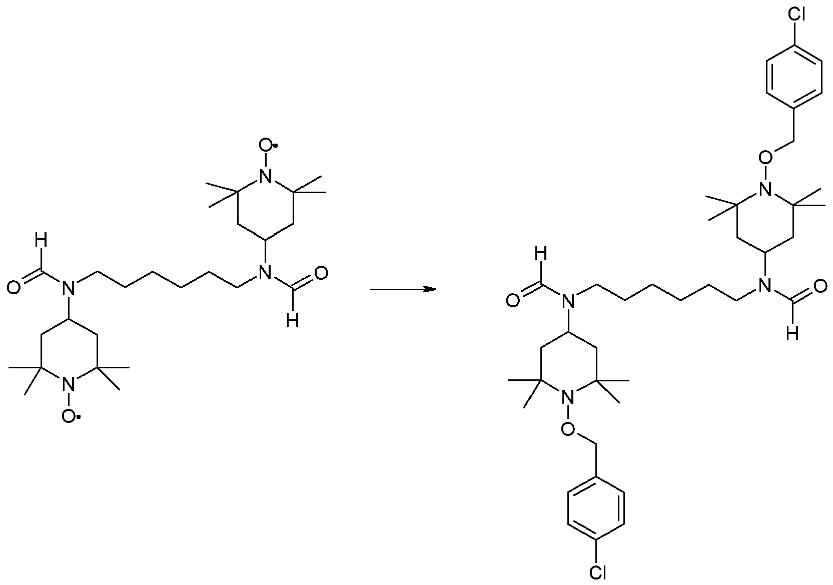

Zu einer entgasten von Mischung von Nitroxylradikal aus Beispiel 1 (50 g), Kupferpulver (16.5 g), Cu(OTf)2 (750 mg mg) und dTbPy (1.95 g) in Toluol (150 mL) wurden innerhalb von 5.5 Stunden bei 75°C 4-Chlor-Benzylbromid (53.3 g) zudosiert und weitere 2 Stunden bei 75°C gerührt. Nach Abkühlen auf Raumtemperatur wurde über Aluminiumoxid vom Rückstand abfiltriert, mit Toluol nachgewaschen, das Filtrat mit 250 ml 25%igem Ammoniakwasser gewaschen, das Filtrat wurde eingeengt und mittels Flash-Chromatographie über Kieselgel gereinigt (Eluent Toluol/Aceton). Man erhielt das Produkt als weißen Feststoff. ¹H NMR (300 M Hz, CDCl₃) δ 1.24-1.34 (m, 30 H), 1.62 (s, 6 H), 1.81 (dd, *J* = 9.6, 22.8 Hz, 2 H), 3.23-3.40 (m, 4 H), 3.60-3.67 (m, 1.4 H), 4.51-4.56 (m, 0.7 H), 4.82 (s, 4 H), 7.27-7.36 (m, 8 H), 8.10-8.18 (m, 2 H).

### Beispiel 3:

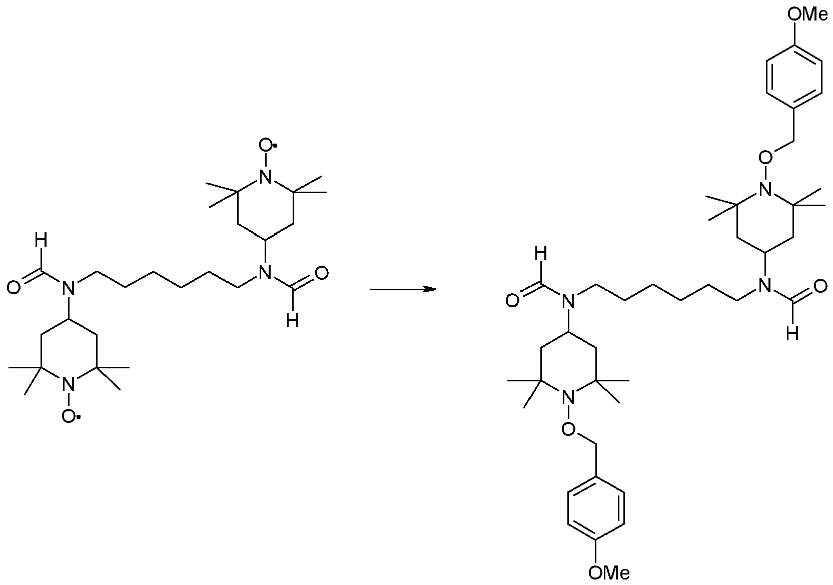

Zu einer entgasten von Mischung von Nitroxylradikal aus Beispiel 1 (10 g), Kupferpulver (3.3 g), Cu(OTf)2 (150 mg mg) und dTbPy (0.39 g) in Toluol (30 mL) wurden innerhalb von 6 Stunden bei 75°C 4-Methoxy-Benzylbromid (10.4 g) zudosiert und weitere 20 Stunden bei 75°C gerührt. Nach Abkühlen auf Raumtemperatur wurde über Aluminiumoxid vom Rückstand abfiltriert, mit Toluol nachgewaschen, das Filtrat mit 250 ml 25%igem Ammoniakwasser gewaschen, das Filtrat wurde eingeengt und durch Ausrühren mit Methanol gereinigt. Man erhielt das Produkt als weißen Feststoff.

### Beispiel 4:

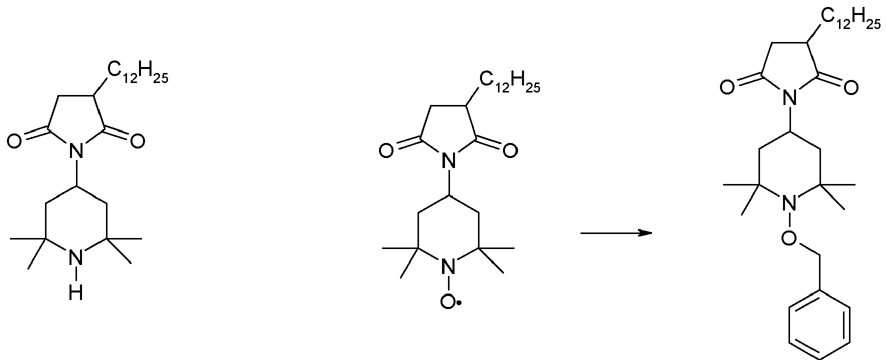

Zu einer Lösung von 3-Dodecyl-1-(2,2,6,6-tetramethyl-piperidin-4-yl)-pyrrolidin-2,5-dion (48.00 g) in MeOH (480 mL) wurden Na2WO4 (9.76 g) und H2O2 (30%, 145 mL) gegeben und 5 Stunden bei Raumtemperatur gerührt. Die Reaktion wurde durch Zugabe von 300 mL gesättigter Thiosulfatlösung gequencht und anschließend mit t-Butylethylether (3x 300 ml) extrahiert. Die vereinten organischen Phasen wurden getrocknet und eingeengt. Man erhielt 46 g (92.5%) als gelbes Öl. Das Produkt wurde ohne weitere Aufreinigung wie folgt weiterverwendet:

Eine Mischung aus Benzylbromid (4.87 g), Nitroxylradikal (12 g), Kupferpulver (1.9 g), Cu(OTf)₂ (100 mg) und dTbPy (0.31 g) in Toluol (90 mL) wurden entgast und für 20 Stunden zum Rückfluss erhitzt. Nach Abkühlen auf Raumtemperatur wurde vom Rückstand abfiltriert. Das Filtrat wurde eingeengt und mittels Flash-Chromatographie über Kieselgel gereinigt (Eluent 1:20 Ethylacetat/Petrolether). Man erhielt 10.6 g (73%) des Produkts als weißen Feststoff. ¹H NMR (300 M Hz, CDCl₃) δ 0.88 (t, *J* = 4.8 Hz, 3 H), 1.25-1.49 (m, 35 H), 1.84-1.92 (m, 1 H), 2.32 (dd, *J* = 2.7, 12.9 Hz, 2 H), 2.51-2.56 (m, 2 H), 2.69-2.79 (m, 2 H), 4.40-4.48 (m, 1 H), 4.84 (s, 2 H), 7.28-7.35 (m, 5 H).

### Beispiel 5:

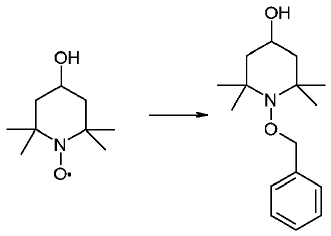

Eine Mischung aus Benzylbromid (12.5 g), 4-Hydroxy-TEMPO (15 g), Kupferpulver ( 4.9 g), Cu(OTf)₂ (260 mg) und dTbPy (0.8 g) in Benzol (90 mL) wurden entgast und für 15 Stunden unter einer Stickstoff-Atmosphäre zum Rückfluss erhitzt. Nach Abkühlen auf Raumtemperatur wurde vom Rückstand abfiltriert. Das Filtrat wurde eingeengt und mittels Umkristallisation aus 1:20 Ethylacetat/Petrolether) gereinigt. Man erhielt 13.5 g (71 %) des Produkts als weißen Feststoff. ¹H NMR (300 MHz, CDCl₃) δ 1.19 (s, 6 H), 1.29 (s, 6 H), 1.49 (d, *J* = 12.3 Hz, 2 H), 1.84 (dd, *J* = 2.4, 12.3 Hz, 2 H), 3.92-4.04 (m, 1 H), 4.82 (s, 2 H), 7.25-7.37 (m, 5 H).

### Beispiel 6:

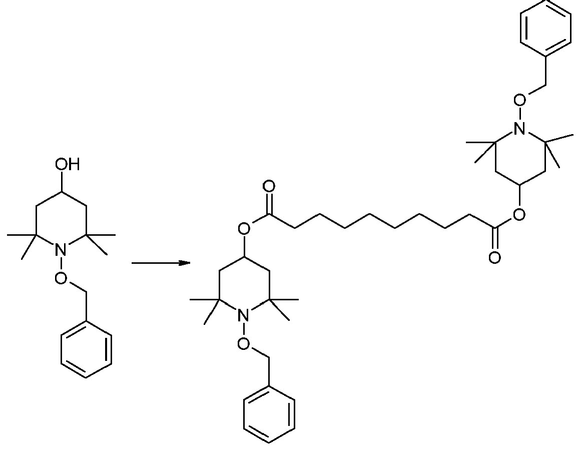

4.5 g Decandisäuredichlorid wurden im Verlauf von 10 min. zu einer gerührten und gekühlten (0°C) Lösung aus 7.8. g der Benzylverbindung aus Beispiel 5, Pyridin (7.1 g) und Dimethylaminopyridin (DMAP,0.8 g) in CH₂Cl₂ (100 mL) getropft. Nach der Zugabe wurde das Kältebad entfernt und die Mischung bei Raumtemperatur für 5 Stunden weitergerührt. Anschließend wurde eine gesättigte Lösung von Ammoniumchlorid zugegeben und dreimal mit je 100 mL Ethylacetat extrahiert. Die organischen Phasen wurden über Natriumsulfat getrocknet und eingeengt. Flash-Chromatographie über Kieselgel (Fließmittel 1:30 Ethylacetat/Petrolether) ergaben 6.2 g des gewünschten Produkts als weißen Feststoff. ¹H NMR (300 M Hz, CDCl₃) δ 1.25-1.30 (m, 34 H), 1.60 (t, *J* = 9.0 Hz, 6 H), 1.85 (d, *J* = 9.9 Hz, 4 H), 2.26 (t, *J* = 7.5 Hz, 4 H), 4.82 (s, 4 H), 5.01-5.08 (m, 2 H), 7.28-7.35 (m, 10 H).

### Beispiel 7:

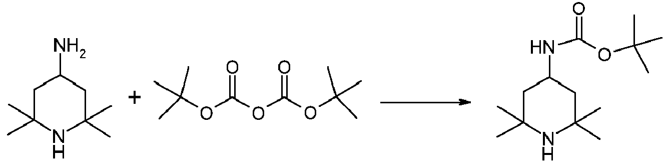

156,3 g(1,0 mol) Triacetondiamin wurden in 2000 ml Dichlormethan vorgelegt und unter Stickstoffatmospähre 90,0 g (0,4 mol) Di-tert.butyldicarbonat 97%ig unter Kühlung bei Raumtemp. zugetropft. Anschliessend wurde 3 Tage bei Raumtemperatur weitergerührt. Zur Reaktionslösung wurden 1000 ml Wasser zugegeben. Die organische Phase wurde zweimal mit je 500 ml Wasser und die wässrige Phase mit 500 ml Dichlormethan extrahiert. Die vereinigten organischen Phasen wurden über Na₂SO₄ getrocknet, und nach dem Abfiltrieren am Rotationsverdampfer vollkommen eingeengt. Man erhielt 91,1 g hellgelbe Kristalle (89 %). Schmp: 180°-181°C

### Beispiel 8:

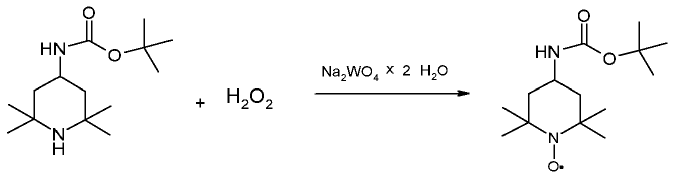

10,1 g (39,4 mmol) der Verbindung aus Beispiel 7 wurden in 250 ml Methanol und 88 ml destilliertem Wasser vorgelegt, 2,2 g (6,6 mmol) Na₂WO₄ x 2 H₂O zugegeben und innerhalb von 10 min. 89,3 g(788 mmol) H₂O₂ 30 %ig bei Raumtemp. zugetropft. Die Mischung wurde bei 60°C 12 Stunden gerührt, anschließend auf Raumtemperatur abgekühlt, abgesaugt und mit Wasser nachgewaschen. Man erhielt 9.5 g orange Kristalle (89% Ausbeute) mit Schmelzpunkt 199° - 200°C.

### Beispiel 9:

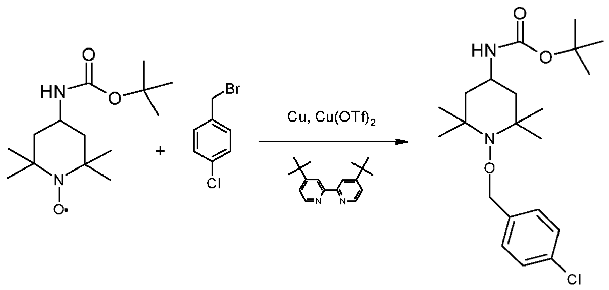

9,0 g (33 mmol) der Verbindung aus Beispiel 8, 2,0 g (32 mmol) Kupferpulver; 0,54 g (1,5 mmol)Cu(OTf)₂ (Kupfertriflat), 1,64 g (6,0 mmol)4,4' - Di - tert.butyl - 2 - 2' dipyridyl 98%ig und 6,2 g(30 mmol) 4 - Chlorbenzylbromid wurden unter Stickstoffatmosphäre in 100 ml Toluol 12 Std. bei 90°C gerührt.Die Reaktionslösung wurde bei Raumtemperatur über Kieselgel abgesaugt, mit Toluol nachgewaschen, zweimal mit je 100 ml 1-2% Ammoniaklösung und 1 mal mit 100 ml Wasser extrahiert. Die organische Phase wurde über Na₂SO₄ getrocknet und nach dem Abfiltrieren im Vakuum vollkommen eingeengt. Man erhielt 5,7 g gelbe Kristalle (48%).

### Beispiel 10:

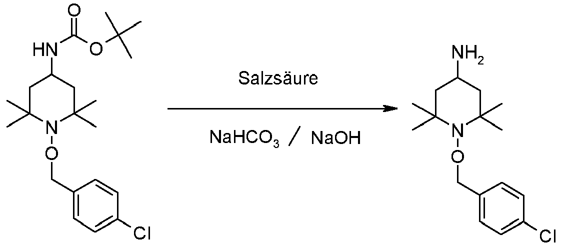

Unter Stickstoffatmosphäre wurden 5,4 g (13,6 mmol) der Verbindung aus Beispiel 9 mit 30 ml Salzsäure (10 %ig) und 50 ml Essigester 2 Stunden bei 90°C gerührt. Nach dem Abkühlen wurden 100 ml gesättigte NaHCO₃ - Lösung und 80 ml 10 %ige NaOH langsam zugegeben (pH 10). Anschließend wurde zweimal mit je 100 ml Essigester und die organische Phase mit 100 ml Wasser extrahiert. Nach dem Trocknen über Na₂SO₄ wurde die organische Phase bei 40°C/10 mbar vollkommen eingeengt. Es wurden 3,4 g beige Kristalle (85%) erhalten.

### Beispiel 11

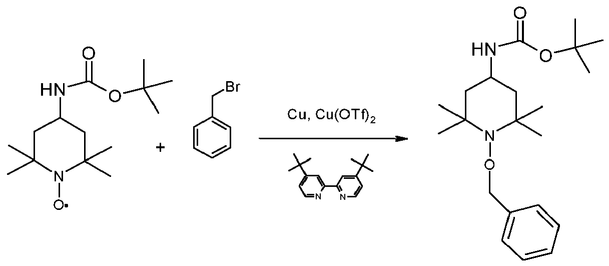

88,1 g (0,325 mol) der Verbindung aus Beispiel 8, 19,5 g (0,315 mol) Kupferpulver, 5,48 g (0,015 mol)Cu(OTf)₂ (Kupfertriflat) ,16,15 g (0,059 mol) 4,4' - Di-tert.butyl - 2 - 2' dipyridyl 98%ig und 56,14 g (0,295 mol) Benzylbromid 98 %ig wurden in 1000 ml Toluol 16 Stunden bei 90°C Innentemperatur gerührt.

Die Reaktionslösung wurde bei Raumtemperatur über Kieselgel abgesaugt, mit Toluol nachgewaschen und am Rotationsverdampfer vollkommen eingeengt. 82,0 g gelbe Kristalle (70%) wurden erhalten.

### Beispiel 12:

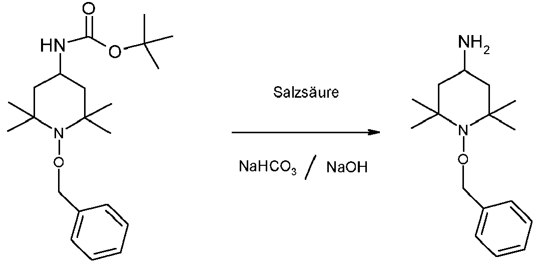

82,0 g (0,226 mol) der Verbindung aus Beispiel 11 wurden in 770 ml Essigester gelöst, bei Raumtemperatur 462 ml Salzsäure 10 %ig zugetropft und die Mischung 2 Stunden bei 90°C gerührt. Nach dem Abkühlen auf Raumtemperatur wurde die Reaktionslösung im Becherglas mit 2000 ml gesättigter NaHCO₃ - Lösung und 237 g NaOH 50 % auf pH 9 alkalisch gestellt. Die Phasen wurden getrennt. Die wässrige Phase wurde zweimal mit je 250 ml Eisessig und die organische Phase mit 250 ml Wasser extrahiert. Nach dem Trocknen über Natriumsulfat und anschließendem Abfiltrieren wurde im Vakuum vollkommen eingeengt. 54,9 g hellbraunes halbkristallines Öl (93%) wurde erhalten.

### Beispiel 13:

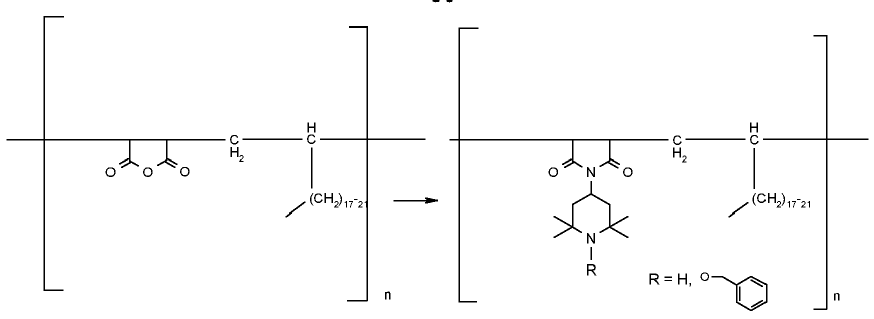

Eine Mischung von 10,3 g der Verbindung aus Beispiel 12 benzyliertem TAD (Triacetondiamin) und 24,5 g Triactenondiamin wurde im 50 g Solvesso 100 (Kohlenwasserstofflösungsmittel mit hohem Anteil an aromatischen Verbindungen) vorgelegt, auf 190°C aufgeheizt und unter Stickstoffatmosphäre 94,5 g eines Coploymeren aus Maleinsäureanhydrid und Olefin (mittlere Molmasse ca. 3500) im 150°C beheizten Tropftrichter innerhalb von 80 min zugetropft. Es wurde 4 h bei 190°C weitergerührt und nach dem Abkühlen wurde das Solvesso am Rotationsverdampfer bei Ölpumpenvakuum 3 mbar und 120°C Badtemperatur eingeengt. Man erhielt 120 g eines glasartigen Rückstands. Onsettemperatur: 400°C

### Vergleichsbeispiel 1:

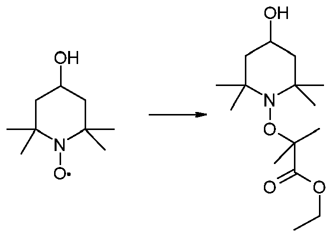

Eine Mischung aus Ethyl-2-Brom-2-methyl-propionat (12.5 g), 4-Hydroxy-TEMPO (20 g), Kupferpulver (7.76 g), Cu(OTf)₂ (420 mg) und dTbPy (1.25 g) in Benzol (120 mL) wurden entgast und für 30 Stunden unter einer Stickstoff-Atmosphäre zum Rückfluss erhitzt. Nach Abkühlen auf Raumtemperatur wurde vom Rückstand abfiltriert. Das Filtrat wurde eingeengt und mittels Säulenchromatographie über mit 1:30 Ethylacetat/Petrolether gereinigt. Man erhielt 4.6 g (15%) des Produkts als weißen Feststoff. ¹H NMR (300 MHz, CDCl₃) δ 1.16 (s, 6 H), 1.21 (s, 6 H), 1.29 (t, *J* = 5.7 Hz, 3 H), 1.41-1.50 (m, 8 H), 1.81 (dd, *J* = 1.2, 3.3 Hz, 2 H), 3.91-3.98 (m, 1 H), 4.17 (dd, *J* = 5.4, 10.8 Hz, 2 H).

### Vergleichsbeispiel 2:

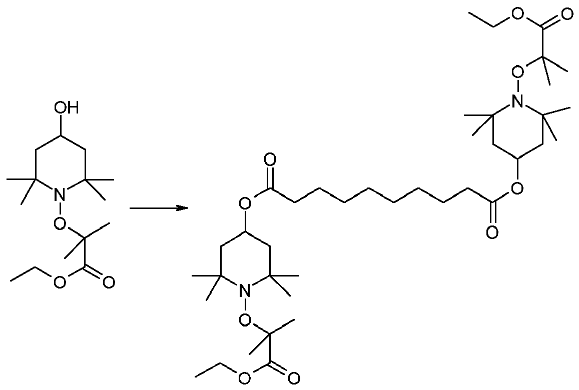

0.88 g Decandisäuredichlorid wurden im Verlauf von 10 min. zu einer gerührten und gekühlten (0°C) Lösung aus 2.1 g der so erhaltenen Verbindung aus Beispiel 7, Pyridin (2.3 g) und Dimethylaminopyridin (DMAP,0.2 g) in CH₂Cl₂ (30 mL) getropft. Nach der Zugabe wurde das Kältebad entfernt und die Mischung bei Raumtemperatur für 16 Stunden weitergerührt. Anschließend wurde eine gesättigte Lösung von Ammoniumchlorid zugegeben und dreimal mit je 50 mL Ethylacetat extrahiert. Die organischen Phasen wurden über Natriumsulfat getrocknet und eingeengt. Flash-Chromatographie über Kieselgel (Fließmittel 1:20 Ethylacetat/Petrolether ergaben 1.75 g des gewünschten Produkts als weißen Feststoff. ¹H NMR (300 MHz, CDCl₃) δ 1.03 (s, 12 H), 1.24-1.31 (m, 26 H), 1.46-1.51 (m, 20 H), 1.55-1.58 (m, 2 H), 1.84 (dd, *J =* 4.2, 12.8 Hz, 4 H), 2.24 (t, *J* = 7.5 Hz, 4 H), 4.16 (dd, *J* = 7.2, 14.4 Hz, 2 H), 4.95-5.06 (m, 2 H).

### Vergleichsbeispiel 3:

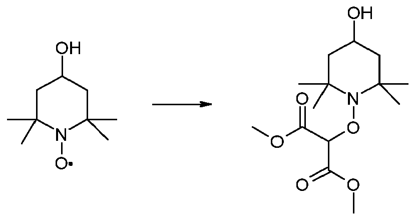

Eine Mischung aus Dimethylbrommalonat (36.60 g), 4-Hydroxy-TEMPO (30 g), Kupferpulver (11.63 g), Cu(OTf)₂ (630 mg) und dTbPy (1.85 g) in Benzol (200 mL) wurden entgast und für 20 Stunden unter einer Stickstoff-Atmosphäre zum Rückfluss erhitzt. Nach Abkühlen auf Raumtemperatur wurde vom Rückstand abfiltriert. Das Filtrat wurde eingeengt und mittels Säulenchromatographie über mit 1:10 Ethylacetat/Petrolether gereinigt. Man erhielt 9.1 g (19%) des Produkts als weißen Feststoff. ¹H NMR (300 MHz, CDCl₃) δ 1.08 (s, 6 H), 1.24 (s, 6 H), 1.43 (t, *J* = 9.3 Hz, 2 H), 1.65 (d, *J* = 3.3 Hz, 1 H), 1.80 (dd, *J=* 1.8, 8.1 Hz, 2 H), 3.77 (s, 6 H), 3.91-3.99 (m, 1 H).

### Vergleichsbeispiel 4:

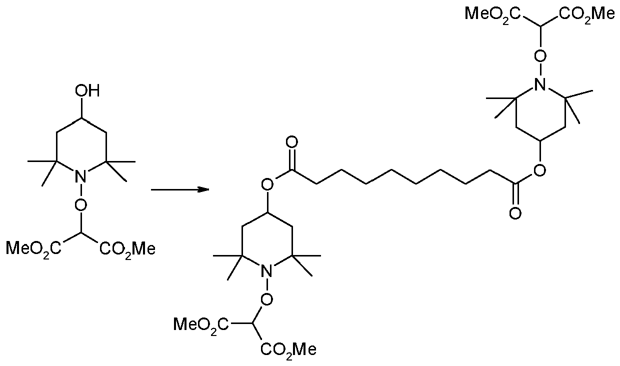

2.5 g Decandisäuredichlorid wurden im Verlauf von 10 min. zu einer gerührten und gekühlten (0°C) Lösung aus 6 g der so erhaltenen Verbindung aus Beispiel 9, Pyridin (6.25 g) und Dimethylaminopyridin (DMAP, 0.5 g) in CH₂Cl₂ (80 mL) getropft. Nach der Zugabe wurde das Kältebad entfernt und die Mischung bei Raumtemperatur für 15 Stunden weitergerührt. Anschließend wurde eine gesättigte Lösung von Ammoniumchlorid zugegeben und dreimal mit je 100 mL Ethylacetat extrahiert. Die organischen Phasen wurden über Natriumsulfat getrocknet und eingeengt. Flash-Chromatographie über Kieselgel (Fließmittel 1:20 Ethylacetat/Petrolether ergaben 3.4 g (46%) des gewünschten Produkts als weißen Feststoff. ¹H NMR (300 MHz, CDCl₃) δ 1.09 (s, 12 H), 1.23-1.29 (m, 20 H), 1.47-1.57 (m, 8 H), 1.83 (dd, *J* = 3.0, 6.3 Hz, 4 H), 2.23 (t, *J* = 5.7 Hz, 4 H), 3.78 (s, 12 H), 4.94 (s, 2 H), 4.97-5.03 (m, 2 H).

### Vergleichsbeispiel 5:

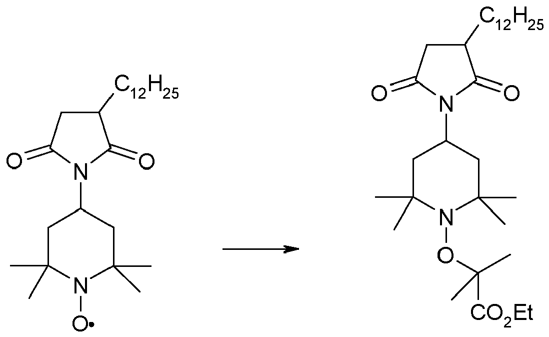

Eine Mischung aus Ethyl-2-brom-2-methyl-propionat (6.91 g), Nitroxylradikal aus Beispiel 4 (15.00 g), Kupferpulver (2.38 g), Cu(OTf)₂ (130 mg) und dTbPy (0.38 g) in 2-Ethoxyethanol (100 mL) wurden entgast und für 5 Stunden unter einer Stickstoff-Atmosphäre auf 70°C erhitzt. Nach Abkühlen auf Raumtemperatur wurde vom Rückstand abfiltriert. Das Filtrat wurde eingeengt und mittels Säulenchromatographie über mit 1:100 Ethylacetat/Petrolether gereinigt. Man erhielt 1.81 g (11 %) des Produkts als weißen Feststoff. ¹H NMR (300 MHz, CDCl₃) δ 0.85 (d, *J =* 3.2 Hz, 2 H), 0.86 (d, *J =* 10.6 Hz, 2 H), 1.12 (s, 6 H), 1.24-1.46 (m, 31 H), 1.46 (s, 6 H), 1.82-1.87 (m, 1 H), 2.29 (dd, *J =* 3.0, 13.2 Hz, 1 H), 2.44 (t, *J =* 9.3 Hz, 2 H), 2.63-2.77 (m, 2 H), 4.15 (dd, *J* = 5.4, 10.8 Hz, 2 H), 4.35-4.42 (m, 1 H).

### Beispiel 14:

Squalan-Test zur Simulation von Polymerstabilisierung unter thermooxidativen Bedingungen

In einem Lagerexperiment wurde Squalan, das als flüssiger Polyolefinerssatzstoff diente, bei 75°C in Gegenwart des Radikalstartes "BUT" [2,2,-Di(tert-butylperoxy)butan] oxidativ abgebaut. Dies wurde GC-chromatographisch oder Ramanspektroskopisch verfolgt. Der ausgewertete Raman-Spektralbereich lag bei 3290 cm⁻¹ bis 2560 cm⁻¹ (CH-Bereich) und bei 1530 cm⁻¹ bis 700 cm⁻¹.

Durch den Zusatz von Stabilisatoren war es möglich, den Abbau von Squalan zu verhindern bzw. zu verlangsamen. Der durch die Verwendung eines Stabilisators verlangsamte Abbau erlaubte die Beurteilung der Wirksamkeit dieser Verbindungen.

Im Experiment wurden 3 g Squalan (7.1 mmol), 0.06 g einer "BUT"-Lösung (50%ige Lösung, 0.3 mmol "BUT") sowie die zu untersuchende Verbindung (0.15 Gew.-%) in einem Reagenzglas vorgelegt. Die resultierende Lösung bzw. Suspension wurde unter Schütteln auf 75°C erhitzt und der Abbau mittels GC-Kontrolle oder Ramanspektroskopisch verfolgt.

**Tabelle 1: Squalangehalt nach einer Anzahl von Tagen [d] in % des Anfangswertes**

| Squalangehalt nach | 2 d | 5 d | 9 d | 12 d | 15 d | 22 d | 29 d | 36 d | 43 d | 50 d | 56 d |
|---|---|---|---|---|---|---|---|---|---|---|---|
| Bsp. 1 | 95,3 | 95,6 | 97,5 | 95,5 | 96,1 | 95,1 | 95,5 | 95,1 | 96,7 | 95,7 | 96,6 |
| VS1 | 95,1 | 95,2 | 94,3 | 92,5 | 92,1 | 91,0 | 89,1 | 88,4 | 86,9 | 88,0 | 87,1 |
| REF | 98,1 | 91,6 | 87,1 | 76,4 | 72,3 | 69,2 | 58,7 | 48,6 | 47,1 | 39,4 | 34,9 |

Vergleichssubstanz (VS1): Decandisäure-bis-(2,2,6,6-tetramethyl-1,octyloxy-piperidin-4-yl)ester (nicht erfindungs-gemäss, Tinuvin® 123)

Referenz ohne Stabilisator (REF): Squalan mit BUT

### Beispiel 15:

Squalan-Test zur Simulation von Polymerstabilisierung unter thermooxidativen Bedingungen in Gegenwart von Agrochemikalien

In einem Lagerexperiment wurde Squalan, das als flüssiger Polyolefinerssatzstoff diente, bei 75°C in Gegenwart des Radikalstartes "BUT" [2,2,-Di(tert-butylperoxy)butan] oxidativ abgebaut. Dies wurde GC-chromatographisch oder Ramanspektroskopisch verfolgt. Der ausgewertete Raman-Spektralbereich lag bei 3290 cm⁻¹ bis 2560 cm⁻¹ (CH-Bereich) und bei 1530 cm⁻¹ bis 700 cm⁻¹.

Durch den Zusatz von Stabilisatoren war es möglich, den Abbau von Squalan zu verhindern bzw. zu verlangsamen. Der durch die Verwendung eines Stabilisators verlangsamte Abbau erlaubte die Beurteilung der Wirksamkeit dieser Verbindungen. In Gegenwart von Agrachemikalien wurde eine verringerte Schutzwirkung der Stabilisatoren beobachtet, die jedoch bei den erfindungsgemässen Verbindungen deutlich geringer ausfällt.

Jeweils 0,0045g (0,15%) des Stabilisators, und 0,0045g (0,15%) der Agrochemikalie wurden in ein randloses Reagenzglas eingewogen und mit 3g einer Lösung aus 2% 2,2-Bis(tert-butylperoxy)-butan (50wt.% in Mineralöl) in Squalan aufgefüllt.

Die resultierende Lösung bzw. Suspension wurde unter Schütteln auf 75°C erhitzt und der Abbau mittels GC-Kontrolle oder Ramanspektroskopisch nach 43 Tagen ermittelt.

| | Bsp. 1 | VS2 | VS3 | VS4 |
|---|---|---|---|---|
| Squalangehalt ohne Agrochemikalie | 96,7 | 92,3 | 87,2 | 90,8 |
| Metiram® (CAS 9006-42-2) | 87,4 | 63,6 | n.a. | 56,9 |
| Mancozeb® (CAS 8018-01-7) | 96,3 | 86,9 | 80,4 | 82,5 |

| | | | | |
|---|---|---|---|---|
| Vergleichssubstanz (VS2): (nicht erfindungs-gemäss, Chimassorb® 119, CAS 106990-43-6) Vergleichssubstanz (VS3): (nicht erfindungs-gemäss, Chimassorb ® 944, CAS 71878-19-8) Vergleichssubstanz (VS4): (nicht erfindungs-gemäss, Tinuvin® NOR 371, CAS2800-4000) | | | | |

### Beispiel 16:

Onsettemperatur: Die Temperaturstabilität der Verbindungen wurde durch Thermogravimetriemessung in einem Platintiegel unter Luft bestimmt. Dazu wurden jeweils Proben in einen Tiegel eingewogen, mit einer Aufheizrate von 10°C/min auf 600°C erhitzt und der Gewichtsverlust bestimmt. Die Onsettemperatur beschreibt den Beginn der Gewichtsabnahme.

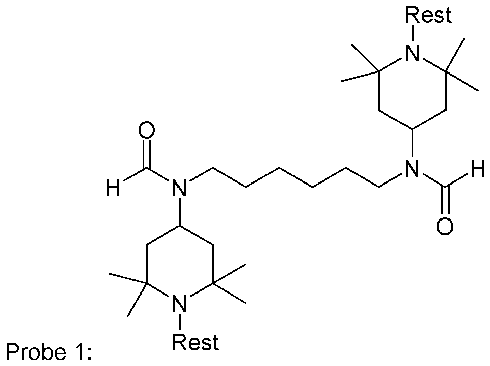

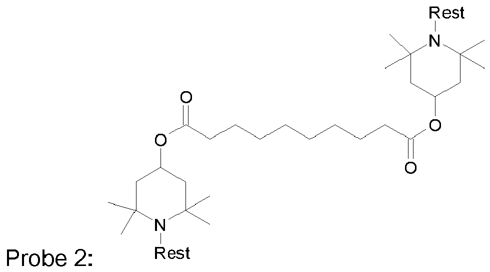

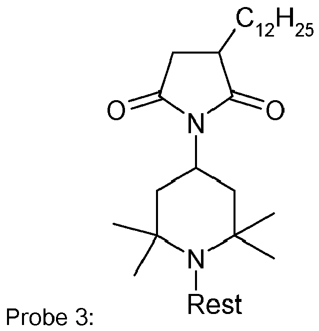

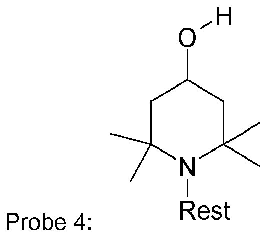

| Rest= | Probe 1 | Probe 2 | Probe 3 | Probe 4 |
|---|---|---|---|---|
| 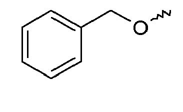 | 258 | 253 | 255 | 208 |
| 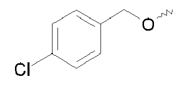 | 256 | nicht bestimmt | nicht bestimmt | nicht bestimmt |
| 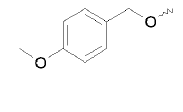 | 247 | nicht bestimmt | nicht bestimmt | nicht bestimmt |
| 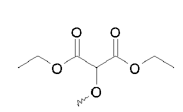 | nicht bestimmt | nicht bestimmt | 219 | 187 |
| (Vergleichsversuch) | | | | |
| 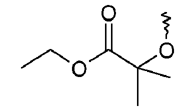 | nicht bestimmt | 152 | 151 | 142 |
| (Vergleichsversuch) | | | | |

### Beispiel 17:

Herstellung einer erfindungsgemäßen LDPE Folie

1000 g Lupolen 1840 D (LDPE, Basell) und 3 g der Verbindung aus Beispiel 1 wurden innig gemischt und mittels eines Zweischneckenextruders extudiert. Das so erhaltene Compound wurde anschließend mit einer Blasfolienanlage zu einer 100µm-dicken Blasfolie verarbeitet.

### Vergleichsbeispiel 6:

Herstellung einer nicht-erfindungsgemäßen LDPE Folie

Analog zu Beispiel wurde eine Folie hergestellt, die jedoch statt der Verbindung aus Beispiel 1 3 g N,N'-Bisformyl-N,N'-bis-(2,2,6,6-tetramethyl-4-piperidinyl)-hexamethylendiamin (CAS- Nr. 124172-53-8, Uvinul® 4050 H, BASF SE) enthielt.

### Beispiel 18:

Künstliche Bewitterung der LDPE-Folien Die Folien aus den Beispiel 9 und Vergleichsbeispiel 6 wurden gemäß der Norm DIN EN ISO 4892-2 künstlich bewittert. Gemessen wurde die Zeit bis zur Versprödung der Folien. Die Ergebnisse sind in der folgenden Tabelle dargestellt:

| | Stabilisator | Zeit bis zur Versprödung |
|---|---|---|
| 10a Folie aus Beispiel 9 | Verbindung aus Beispiel 1 | >14.400 h |
| 10b Folie aus Vergleichsbeispiel 6 | N,N'-Bisformyl-N,N'-bis-(2,2,6,6-tetramethyl-4-piperidinyl)-hexamethylendiamin | 2.000 h |

## Patentansprüche

1. Verwendung eines sterisch gehinderten Amins der allgemeinen Formeln (I-a), (I-c) bis (I-t) und (I-b-1) zur Stabilisierung von unbelebtem organischen Material; 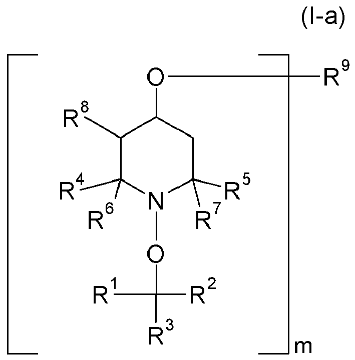 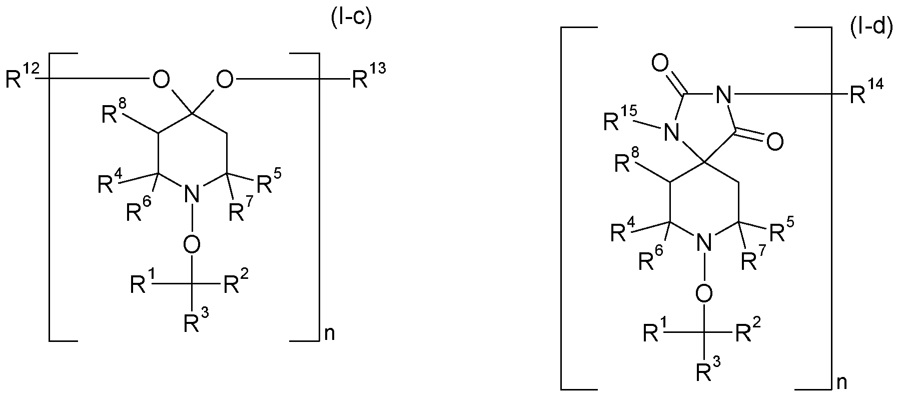 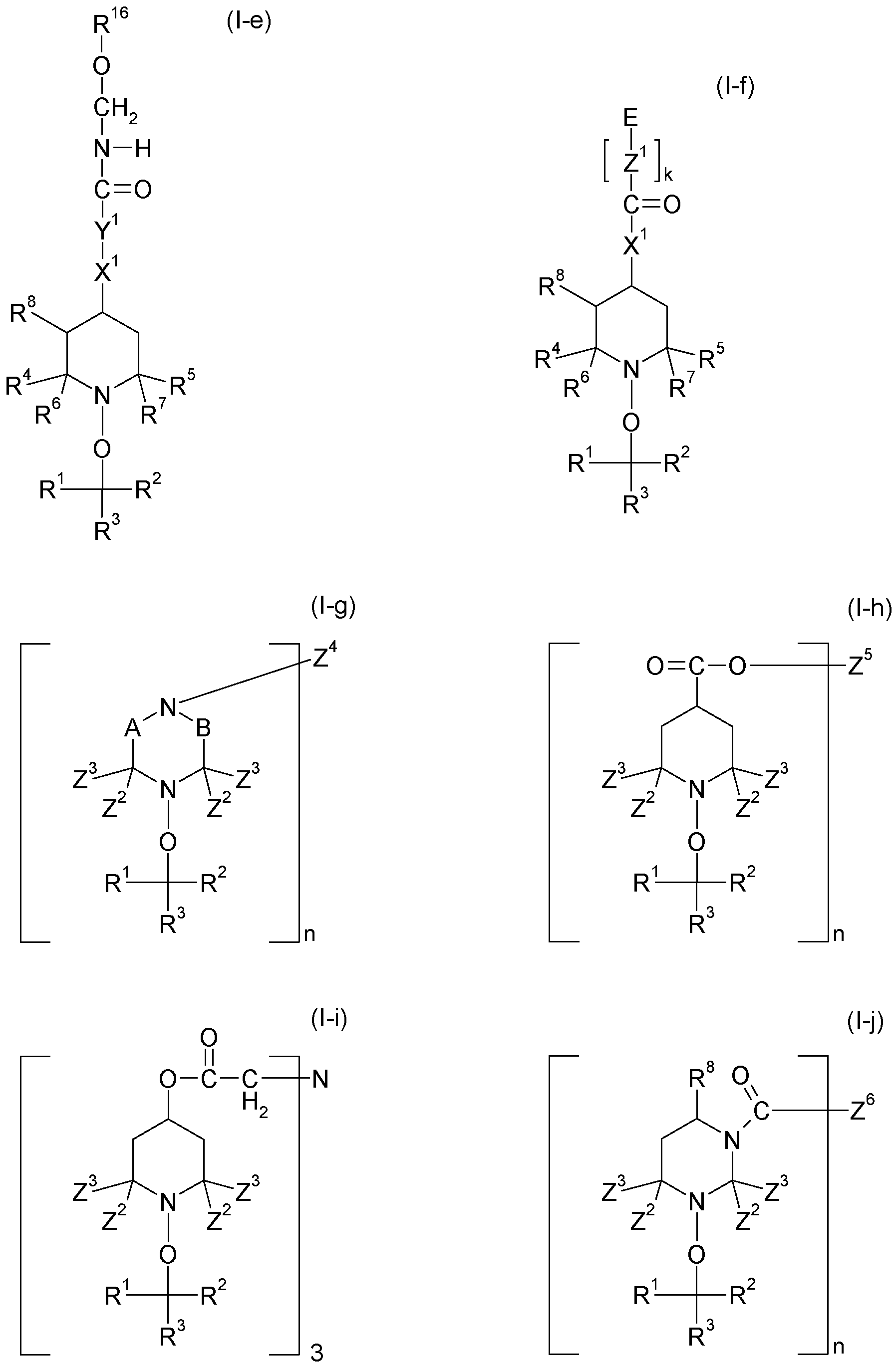 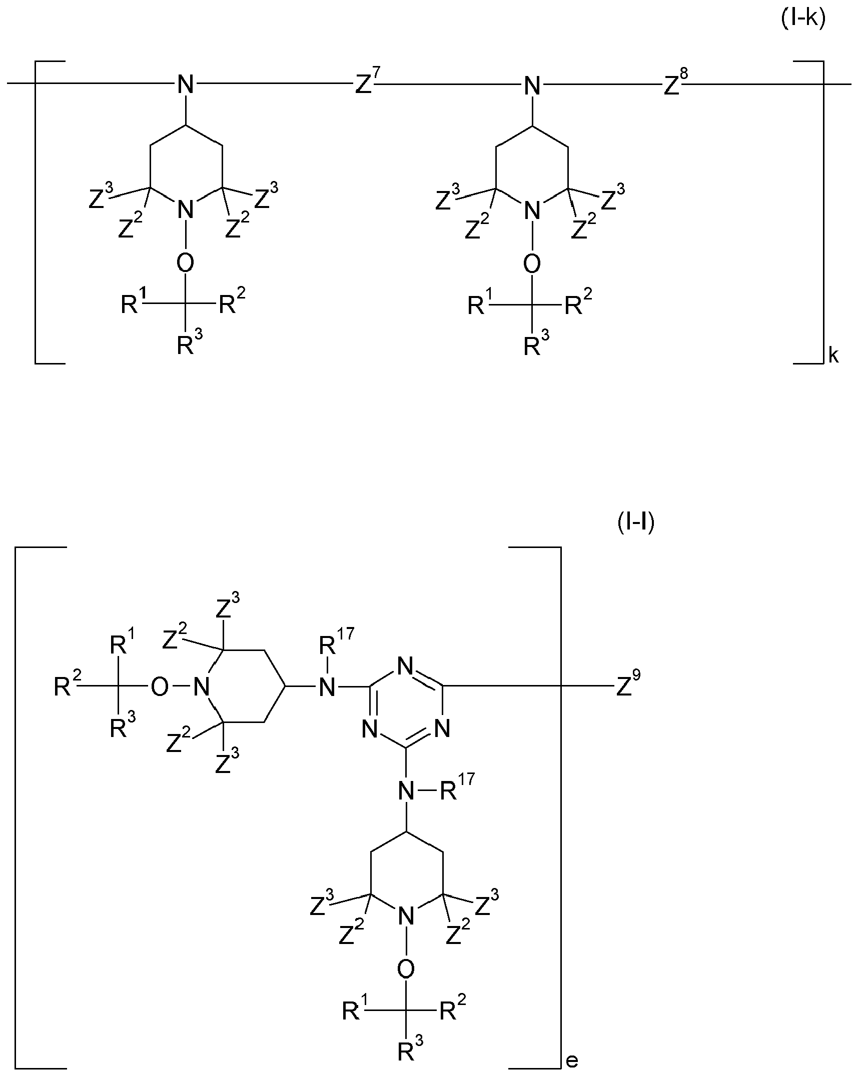 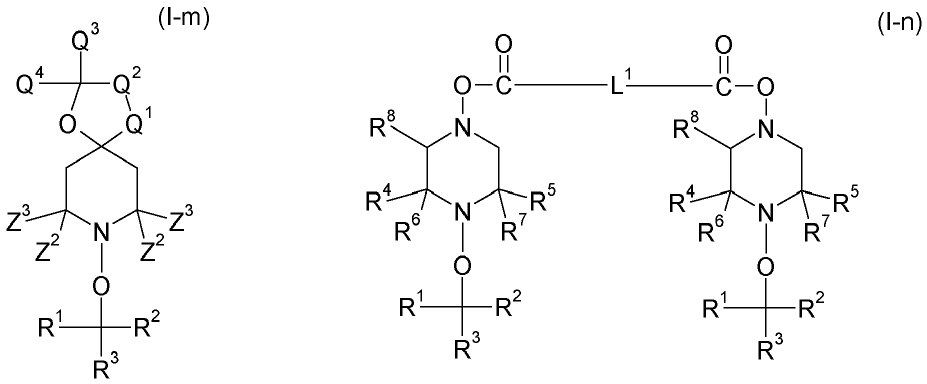 (I-o) - Kondensationsprodukt von (I-m) mit Epichlorhydrin (symbolisch): 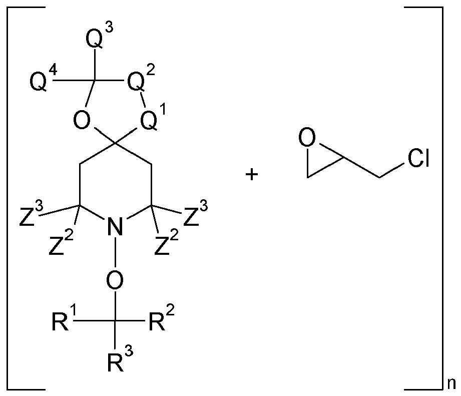 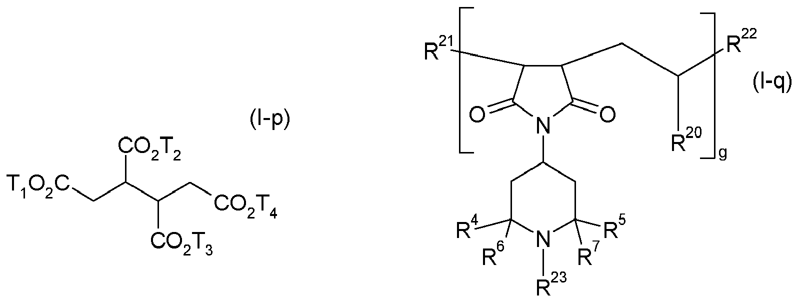 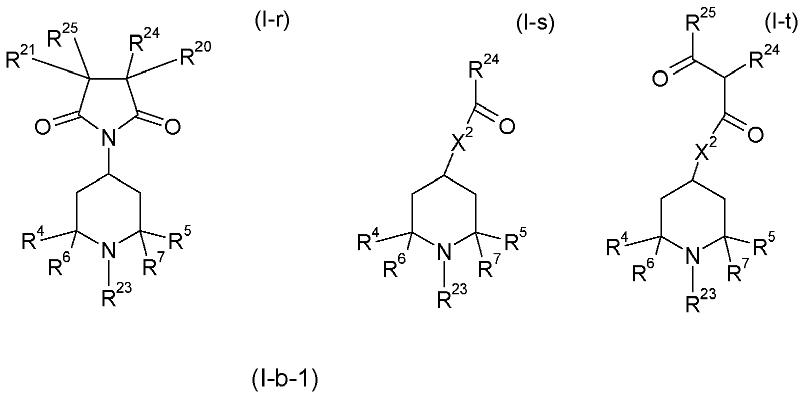 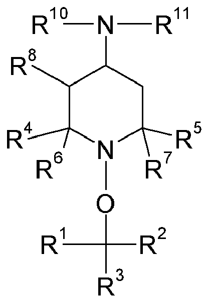 wobei,
R¹, R² unabhängig voneinander, gleich oder verschieden, H, C₁-C₂₀-Alkyl, C₃-C₁₅-Cycloalkyl, Aryl, Hetaryl, Heterocyclus,
R⁴, R⁵, R⁶, R⁷ unabhängig voneinander, gleich oder verschieden, C₁-C₂₀-Alkyl, oder R⁴ und R⁶ oder R⁵ und R⁷ zusammen eine Tetramethylen- oder Pentamethylengruppe
sind, und
R³ Aryl, Hetaryl
ist, und
wobei R¹ bis R⁷ jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch Hydroxy, Amino, Mono- oder Di-C₁-C₂₀-Alkylamino, Nitro, Cyano, CO₂M¹, CONM¹M², SO₃M¹, C₁-C₂₀-Alkyl, C₁-C₂₀-Alkoxy, Aryl, Heterocyclus, Heteroatome oder Halogen substituiert sein können, wobei diese ebenfalls maximal zweimal, bevorzugt maximal einmal mit den genannten Gruppen substituiert sein können und
M¹, M² unabhängig voneinander, gleich oder verschieden, H, C₁-C₂₀-Alkyl sind,
mit
R⁸ H, CH₃,
R¹⁰ H, C₁-C₂₀-Alkyl, C₃-C₁₅-Cycloalkyl, C₇-C₉-Aralkyl, C₁-C₂₁-Alkanoyl, Benzoyl,
R¹⁵ H, C₁-C₂₀-Alkyl, Allyl, Benzyl, Glycidyl, C₁-C₂₀-Alkoxyalkyl,
X¹ -N(R¹⁸)-, -O-,
R¹⁸ H, C₁-C₂₀-Alkyl, C₃-C₁₅-Cycloalkyl, C₇-C₁₂-Aralkyl, Cyanoethyl, Aryl, -CH₂-CH(R¹⁹)-OH, eine Gruppe der allgemeinen Formel (II), (III), (IV) oder (V), 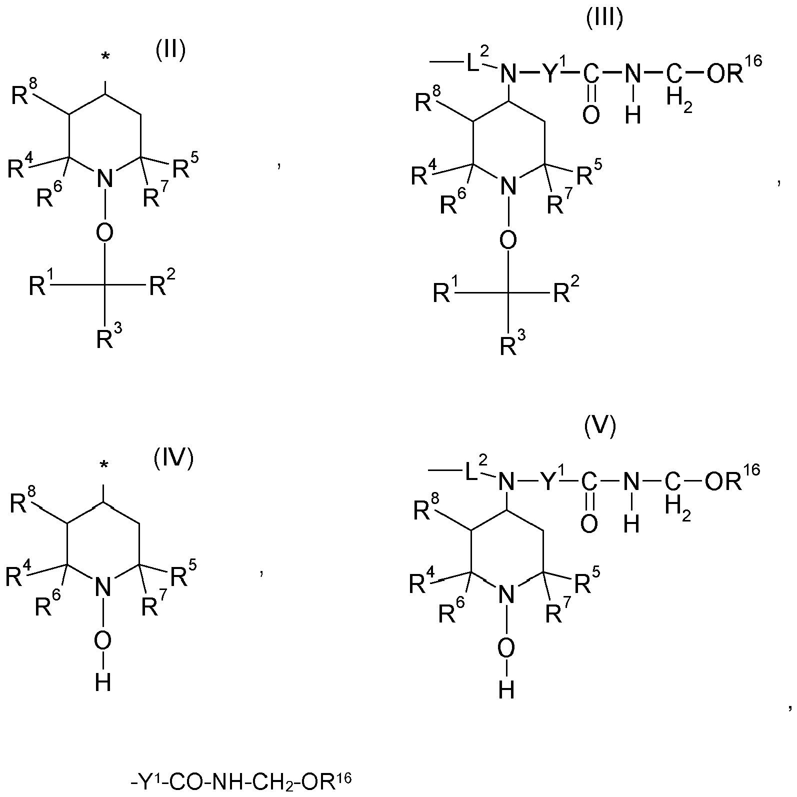
R¹⁹ H, CH₃, Phenyl,
R¹⁶ H, C₁-C₂₀-Alkyl, C₂-C₂₀-Alkenyl, Aryl, Hetaryl
Y¹ C₁-C₃-Alkylen, -CH₂-CH(R¹⁹)-O-, -(CH₂)₃-NH-, Einfachbindung,
L² C₂-C₆-Alkylen, Arylen,
A, B unabhängig voneinander, gleich oder verschieden, Methylen, Carbonyl,
m 1,2,3,4,
und
falls m =1 ist,
R⁹ H, C₁-C₂₀-Alkyl, C₂-C₂₀-Alkenyl, Aryl, C₇- C₂₀-Aralkyl, Glycidyl, C₂-C₂₂-Acyl, eine Gruppe der allgemeinen Formel (VI) oder (VII) 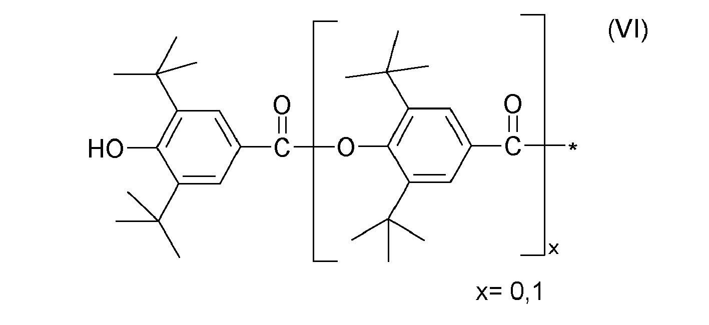 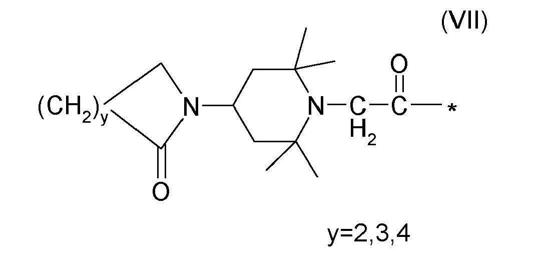
und
falls m = 2 ist,
R⁹ C₁-C₂₀-Alkylen, C₂-C₂₀-Alkenylen, Xylylen, C₂-C₂₀-Di-acyl, eine Gruppe der allgemeinen Formel (VIII) oder (IX) 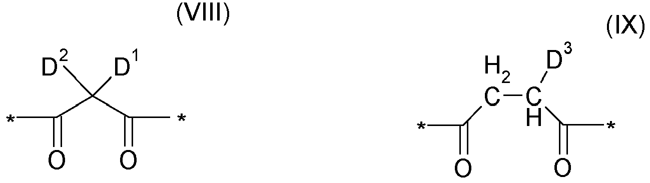
D¹, D² unabhängig voneinander, gleich oder verschieden, H, C₁-C₂₀-Alkyl, Phenyl, Benzyl, 3,5-Di-t-butyl-4-hydroxy-benzyl oder D¹ und D² zusammen C₃-C₆-Alkylen,
D³ C₁-C₂₀-Alkyl, C₂-C₂₀-Alkenyl,
und
falls m = 3 ist,
R⁹ C₄-C₂₀-Tri-acyl,
und
falls m = 4 ist,
R⁹ C₅-C₂₀-Tetra-acyl
und
R¹¹ H, C₁-C₂₀-Alkyl, C₃-C₁₅-Cycloalkyl, C₂-C₂₀-Alkenyl, C₁-C₂₁-Alkanoyl, Carbonyl, Carbamid, Aryl, C₇-C₁₀-Aralkyl, Glycidyl, -CH₂-CH(OH)-H, -CH₂-CH(OH)-CH₃, -CH₂-CH(OH)-Phenyl, eine Gruppe der allgemeinen Formel (II) oder (IV), oder R¹⁰ und R¹¹ bilden gemeinsam ein C₄-C₆-Alkylen oder 1-Oxoalkylen, oder ein C₂-C₂₀-Di-alkanoyl;
n 1,2
und falls n = 1 ist,
R¹² C₁-C₂₀-Alkyl, C₂-C₂₀-Alkenyl, C₇-C₁₂-Aralkyl,
R¹³ H, C₁-C₂₀-Alkyl, C₂-C₂₀-Alkenyl, C₇-C₁₂-Aralkyl, oder R¹³ bildet gemeinsam mit R¹² ein C₂-C₈-Alkylen, C₂-C₈-Hydroxyalkylen, C₄-C₂₂-Acyloxyalkylen,
R¹⁴ H, C₁-C₂₀-Alkyl, C₃-C₅-Alkenyl, C₇-C₉-Aralkyl, C₃-C₁₅-Cycloalkyl, C₂-C₄-Hydroxyalkyl, C₂-C₆-Alkoxyalkyl, Aryl, Glycidyl, -(CH₂)ₜ-COO-Q, -(CH₂)ₜ-O-CO-Q, mit t = 1, 2 und Q = C₁-C₄-Alkyl, Phenyl,
und
falls n = 2 ist,
R¹², R¹³ (-CH₂)₂C(CH₂-)₂,
R¹⁴ C₂-C₂₀-Alkylen, Arylen, -CH₂CH(OH)-CH₂-O-X-CH₂-CH(OH)-CH₂-, mit X = C₂-C₁₀-Alkylen, Arylen, C₆-C₁₂-Cycloalkylen, oder -CH₂CH(OZ')-CH₂-(O-CH₂-CH(OZ')CH₂)₂, mit Z' = H, C₁-C₂₀-Alkyl, Allyl, Benzyl, C₂-C₁₂-Alkynoyl, Benzoyl,
Z¹ Ethylen, 1,2-Propylen, Wiederholungseinheit eines Polymers oder Copolymers abgeleitet von einem oder mehreren Alpha-Olefinen,
k eine ganze Zahl aus dem Bereich von 2 bis 100,
E H, C₁-C₂₀-Alkyl, Aryl, C₁-C₂₀-Alkoxy, C₂-C₂₀-Alkenyl,
Z⁴ H, C₁-C₂₀-Alkyl, C₃-C₁₅-Cycloalkyl, C₂-C₂₀-Alkenyl, C₁-C₂₁-Alkanoyl, Carbonyl, Carbamid, Aryl, C₇-C₁₀-Aralkyl, Glycidyl, -CH₂-CH(OH)-H, -CH₂-CH(OH)-CH₃, -CH₂-CH(OH)-Phenyl, eine Gruppe der allgemeinen Formel (II) oder (IV)
oder
Z⁴ C₁-C₁₂-Alkylen, Xylylen, --CH₂-CH(OH)-CH2-, O-(C₂-C₁₀-Alkylen)-O-CH₂-CH(OH)-CH₂-, Arylen, C₆-C₁₂-Cycloalkylen, Z⁴ kann auch ein C₂-C₂₀-Di-acyl sein, oder -CO-, eine Gruppe der allgemeinen Formel (X) 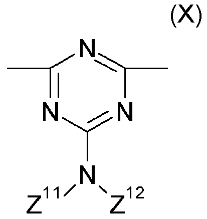 mit Z¹¹, Z¹² unabhängig voneinander, H, C₁-C₂₀-Alkyl, eine Gruppe der allgemeinen Formel (II), oder Z¹¹ und Z¹² bilden gemeinsam ein C₄-C₆-Alkylen oder 3-Oxapentamethylen,
Z² Methyl,
Z³ Methyl, Ethyl, oder Z³ und Z² bilden gemeinsam eine Tetramethylen oder Pentamethylengruppe,
Z⁵ die gleiche Bedeutung wie R¹⁴,
Z⁷, Z⁸ unabhängig voneinander, gleich oder verschieden, C₂-C₁₂-Alkylen, oder Z⁷ ist eine Gruppe der allgemeinen Formel (VIII),
e 2,3,4,
Z⁹ -N(R¹³)-(CH₂)_{d}-N(R¹³)-, oder eine Gruppe der allgemeinen Formel (XI) 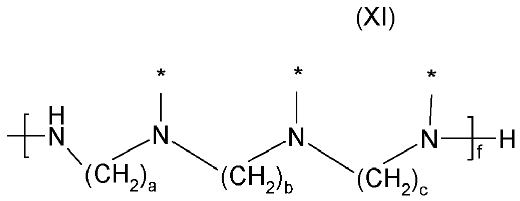 mit a, b, c unabhängig voneinander, gleich oder verschieden 2 oder 3 und d eine ganze Zahl aus dem Bereich von 2 bis 10 und f = 0 oder 1,
R¹⁷ die gleiche Bedeutung wie R¹⁰,
Z⁶ H, C₁-C₂₀-Alkyl, C₃-C₁₅-Cycloalkyl, C₂-C₂₀-Alkenyl, C₁-C₂₁-Alkanoyl, Carbonyl, Carbamid, Aryl, C₇-C₁₀-Aralkyl, Glycidyl, -CH₂-CH(OH)-H, -CH₂-CH(OH)-CH₃, -CH₂-CH(OH)-Phenyl, eine Gruppe der allgemeinen Formel (II) oder (IV)
oder
Z⁶ C₁-C₁₂-Alkylen, Xylylen, --CH₂-CH(OH)-CH2-, O-(C₂-C₁₀-Alkylen)-O-CH₂-CH(OH)-CH₂-, Arylen, C₆-C₁₂-Cycloalkylen, Z⁶ kann auch ein C₂-C₂₀-Di-acyl sein, oder -CO-, eine Gruppe der allgemeinen Formel (X) 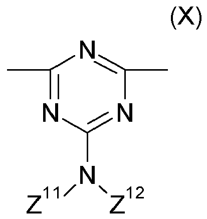 mit Z¹¹, Z¹² unabhängig voneinander, H, C₁-C₂₀-Alkyl, eine Gruppe der allgemeinen Formel (II), oder Z¹¹ und Z¹² bilden gemeinsam ein C₄-C₆-Alkylen oder 3-Oxapentamethylen,
Q¹, Q² verschieden voneinander, jeweils -CO-, N(Q⁵)-
Q⁵ H, C₁-C₂₀-Alkyl, C₄-C₂₀-Alkoxycarbonyl,
Q³ H, C₁-C₂₀-Alkyl, Phenyl, Naphthyl,
Q⁴ H, C₁-C₂₀-Alkyl, Phenyl, Naphthyl, C₇-C₁₂-Phenylalkyl, oder Q4 und Q3 bilden gemeinsam ein C₄-C₁₇-Alkylen,
L¹ Einfachbindung, C₁-C₁₂-Alkylen, Phenylen, -NH-(C₁-C₁₂-Alkylen)-NH-
T¹,T²,T³,T⁴ unabhängig voneinander, gleich oder verschieden C₂-C₂₀-Alkyl, oder ein Strukturelement der allgemeinen Formel (II), unter der Maßgabe, dass mindestens einer der Substituenten T¹, T², T³ oder T⁴ einem Strukturelement der allgemeinen Formel (II) entspricht
g eine ganze Zahl aus dem Bereich von 2 bis 1000,
R²⁰ C₁-C₃₀-Alkyl, Aryl, Gemisch verschiedener C₁-C₃₀-Alkyle,
R²¹, R²² unabhängig voneinander, gleich oder verschieden, H, C₁-C₂₀-Alkyl, Aryl, C₁-C₂₀-Alkoxy, C₂-C₂₀-Alkenyl,
R²³ H, O, OH, C₁-C₂₀-Alkyl, -OC(R¹R²R³), unter der Maßgabe, dass mindestens einer der Substituenten R²³ -OC(R¹R²R³) ist,
R²⁴, R²⁵ unabhängig voneinander, gleich oder verschieden, C₁-C₂₄-Alkyl, bevorzugt C₁₀-C₂₄-Alkyl, besonders bevorzugt C₁₄-C₂₂-Alkyl, ganz besonders bevorzugt C₁₈-C₂₂-Alkyl,
X² O, NH
und
wobei R⁹, R¹¹, Q³, Q⁴, Z⁴, Z⁶ jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch C₁-C₂₀-Alkyl, C₁-C₂₀-Alkoxy, Aryl, Heterocyclus, Heteroatome oder Halogen substituiert sein können, wobei diese ebenfalls maximal zweimal, bevorzugt maximal einmal mit den genannten Gruppen substituiert sein können.

2. Verwendung nach Anspruch 1 , wobei die Substituenten R⁴, R⁵, R⁶, R⁷ Methyl sind.

3. Verwendung nach Anspruch 2, wobei R⁸ Wasserstoff ist.

4. Verwendung nach den Ansprüchen 1 bis 3, wobei neben den sterischen gehinderten Aminen zusätzlich UV-Absorber, Antioxidantien, Costabilisatoren, Metalldesaktivatoren, Metallseifen, Weichmacher, antistatische Mittel, Gleitmittel, Trennmittel, Verarbeitungshilfsmittel, Antiblockmittel, Antibeschlagsmittel, Flammschutzmittel, Pigmente, Farbstoffe, IR-Strahlung regulierende Verbindungen, Schäumungsmittel, Nukleierungsmittel und Füllstoffe in einem Gemisch eingesetzt werden.

5. Verwendung nach den Ansprüchen 1 bis 3 zur Stabilisierung von Kunststoffen oder Lacken.

6. Verwendung nach den Ansprüchen 1 bis 3 zur Stabilisierung von Agrarfolien.

7. Verwendung nach den Ansprüchen 1 bis 3 zur Stabilisierung von PVC.

8. Verwendung nach den Ansprüchen 1 bis 3 zur Stabilisierung von flammgeschützten Thermoplasten.

9. Verfahren zur Stabilisierung von unbelebten organischen Materialien, **dadurch gekennzeichnet, dass** diesen Materialien eines oder mehrere der sterisch gehinderten Amine der allgemeinen Formeln (I-a), (I-c) bis (I-t) und (I-b-1) gemäß Anspruch 1 in einer für die Stabilisierung effektiven Menge zugesetzt werden.

10. Sterische gehindertes Amin der allgemeinen Formel (I-q) gemäß Anspruch 1, wobei R²⁰ ein Gemisch aus C₁₈-C₂₂-Alkylgruppen ist und die Mehrzahl der Substituenten R²³ einer Gruppe -OCR¹R²R³ entsprechen mit
R¹, R² unabhängig voneinander, gleich oder verschieden, H, C₁-C₂₀-Alkyl, C₃-C₁₅-Cycloalkyl, Aryl, Hetaryl, Heterocyclus,
R³ Aryl, Hetaryl,
R⁴, R⁵, R⁶, R⁷ unabhängig voneinander, gleich oder verschieden, C₁-C₂₀-Alkyl, oder R⁴ und R⁶ oder R⁵ und R⁷ zusammen eine Tetramethylen- oder Pentamethylengruppe,
R²¹, R²² unabhängig voneinander, gleich oder verschieden, H, C₁-C₂₀-Alkyl, Aryl, C₁-C₂₀-Alkoxy, C2-C20-Alkenyl.

11. Sterische gehindertes Amin der allgemeinen Formel (I-r) gemäß Anspruch 1, wobei
R⁴, R⁵, R⁶, R⁷ unabhängig voneinander, gleich oder verschieden, C₁-C₂₀-Alkyl, oder R⁴ und R⁶ oder R⁵ und R⁷ zusammen
R²³ eine Tetramethylen- oder Pentamethylengruppe, -OC(R¹R²R³)
R¹, R² unabhängig voneinander, gleich oder verschieden, H, C₁-C₂₀-Alkyl, C₃-C₁₅-Cycloalkyl, Aryl, Hetaryl, Heterocyclus,
R³ Aryl, Hetaryl,
R²⁰ C₁-C₃₀-Alkyl, Aryl, Gemisch verschiedener C₁-C₃₀-Alkyl,
R²¹ unabhängig voneinander, gleich oder verschieden, H, C₁-C₂₀-Alkyl, Aryl, C₁-C₂₀-Alkoxy, C₂-C₂₀-Alkenyl,
R²⁴, R²⁵ unabhängig voneinander, gleich oder verschieden, C₁-C₂₄-Alkyl,
X² O, NH
sind.

12. Sterische gehindertes Amin der allgemeinen Formel (I-s) gemäß Anspruch 1, wobei
R⁴, R⁵, R⁶, R⁷ unabhängig voneinander, gleich oder verschieden, C₁-C₂₀-Alkyl, oder R⁴ und R⁶ oder R⁵ und R⁷ zusammen eine Tetramethylen- oder Pentamethylengruppe,
R²³ -OC(R¹R²R³)
R¹, R² unabhängig voneinander, gleich oder verschieden, H, C₁-C₂₀-Alkyl, C₃-C₁₅-Cycloalkyl, Aryl, Hetaryl, Heterocyclus,
R³ Aryl, Hetaryl,
R²⁴ unabhängig voneinander, gleich oder verschieden, C₁-C₂₄-Alkyl
sind.

13. Sterische gehindertes Amin der allgemeinen Formel (I-t) gemäß Anspruch 1, wobei
R⁴, R⁵, R⁶, R⁷ unabhängig voneinander, gleich oder verschieden, C₁-C₂₀-Alkyl, oder R⁴ und R⁶ oder R⁵ und R⁷ zusammen eine Tetramethylen- oder Pentamethylengruppe,
R²³ -OC(R¹R²R³)
R¹, R² unabhängig voneinander, gleich oder verschieden, H, C₁-C₂₀-Alkyl, C₃-C₁₅-Cycloalkyl, Aryl, Hetaryl, Heterocyclus,
R³ Aryl, Hetaryl,
R²⁴, R²⁵ unabhängig voneinander, gleich oder verschieden, C₁-C₂₄-Alkyl,
X² O, NH
sind.

14. Unbelebte organische Materialien enthaltend sterisch gehinderte Amine gemäß den Ansprüchen 10 bis 13.
